(19) Europäisches Patentamt
European Patent Office
Office européen des brevets

(11) EP 0 835 896 A1

(12) **EUROPEAN PATENT APPLICATION**
published in accordance with Art. 158(3) EPC

(43) Date of publication:
**15.04.1998 Bulletin 1998/16**

(21) Application number: **97919661.5**

(22) Date of filing: **23.04.1997**

(51) Int. Cl.$^6$: **C08G 69/44**, C08L 77/12, C07C 233/65, C07C 235/46, D01F 6/82, C08J 5/00, C07C 233/04

(86) International application number:
**PCT/JP97/01411**

(87) International publication number:
**WO 97/41166 (06.11.1997 Gazette 1997/47)**

(84) Designated Contracting States:
**DE FR GB IT**

(30) Priority: **30.04.1996 JP 109990/96**
**30.04.1996 JP 109991/96**
**30.01.1997 JP 17095/97**
**13.02.1997 JP 28887/97**

(71) Applicant:
**TORAY INDUSTRIES, INC.**
**Tokyo 103 (JP)**

(72) Inventors:
- **YAMAUCHI, Kouji**
**Midori-ku, Nagoya-shi, Aichi 458 (JP)**
- **INOUE, Toshihide**
**Ichinomiya-shi, Aichi 491 (JP)**
- **KANOMATA, Akinori**
**Mizuho-ku, Nagoya-shi, Aichi 467 (JP)**

(74) Representative:
**Coleiro, Raymond et al**
**MEWBURN ELLIS**
**York House**
**23 Kingsway**
**London WC2B 6HP (GB)**

(54) **POLYESTER AMIDE COPOLYMER AND PROCESS FOR THE PRODUCTION THEREOF, POLYESTER AMIDE MONOMER AND PROCESS FOR THE PRODUCTION THEREOF, AND POLYESTER AMIDE RESIN COMPOSITION**

(57) A polyester amide copolymer characterized in that the copolymer has a repeated structure substantially represented by general formula (1) and that if mole percentages of three diad sequences present in the polymer and represented by formulas (2), (3) and (4) are X, Y and Z (mol%) ( X + Y + Z = 100 ), the mole percentage of X in the polymer satisfies $70 \leq X \leq 100$ (mol%):

EP 0 835 896 A1

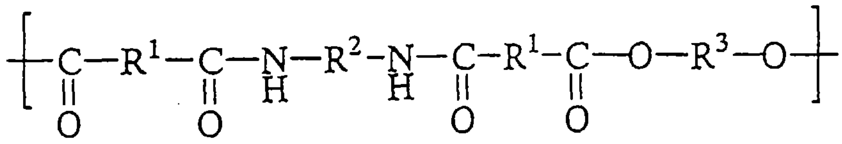

(1)

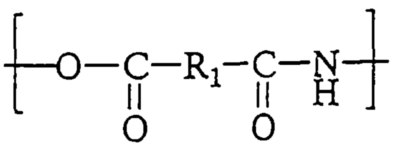

(2)

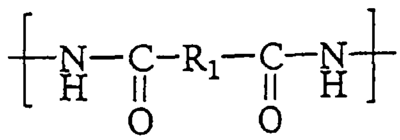

(3)

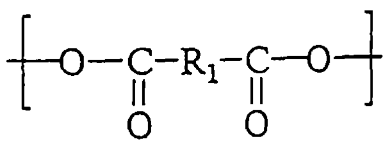

(4)

(where $R^1$, $R^2$ and $R^3$ represent bivalent substituted or non-substituted aliphatic group, alicyclic group or aromatic group).

## Description

### TECHNICAL FIELD

The present invention relates to a high-molecular weight polyester amide copolymer having a structure formed of an ester unit and an amide unit and, further, relates to a polyester amide copolymer excellent in heat stability, heat resistance, processability and crystallinity and useful as an engineering plastic, a fiber and a film, and a method of producing the same, and an ester amide monomer and a method of producing the same, and a resin composition containing the polyester amide copolymer, and a fiber or a film formed from the polyester amide copolymer or the resin composition.

### BACKGROUND ART

To exploit excellent properties of polyester resin and polyester amide resin, methods of copolymerizing an ester unit and an amide unit are proposed.

For example, a method for synthesizing a polyester amide copolymer comprising 1,4-butanediol, 1,4-butanediamine, and terephthalic acid units is disclosed in the description of European Patent Laid-Open No. 445548. Furthermore, J. Polym. Sci., 61,353 (1962) and the specification of United States Patent 2,851,443 report a method for synthesizing a polyester amide copolymer comprising an aliphatic diamine, an aliphatic diol and terephthalic acid.

However, polyester amide copolymers obtained by the methods described in the description of European Patent Laid-Open No. 445548, J. Polym. Sci., 61,353 (1962) and the specification of United States Patent 2,851,443 have low polymer molecular weights and are very poor in crystallinity, heat stability and heat resistance. Therefore, it is difficult to process the copolymers to produce not only resin moldings but also films and fibers. The production of processed fibers and films is especially difficult because polymer decomposition progresses and foaming occurs due to dwell during processes for films and fibers.

It is an object of the present invention to provide a high-purity ester amide monomer synthesized by a specific method, a polyester amide copolymer produced from the ester amide monomer and having a high molecular weight, wherein the sequences of an ester unit and an amide unit repeatedly alternate in the polyamide copolymer, and a fiber, a film or the like incorporating the polyester amide copolymer and being excellent in heat resistance, heat stability, processability and crystallinity.

### DISCLOSURE OF THE INVENTION

Accordingly, the present invention provides the following:

1. A polyester amide copolymer characterized in that the copolymer has a repeated structure substantially represented by general formula (1) and that if mol percentages of three diad sequences present in the polymer and represented by formulas (2), (3) and (4) are X, Y and Z (mol%) ( $X + Y + Z = 100$ ), the mole percentage of X in the polymer satisfies $70 \leq X \leq 100$ (mol%):

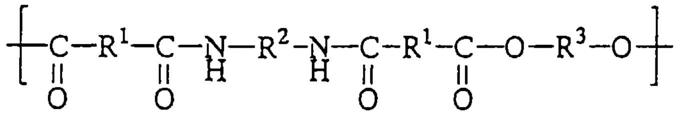

(1)

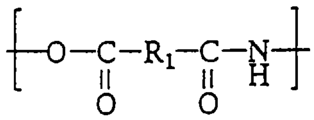

(2)

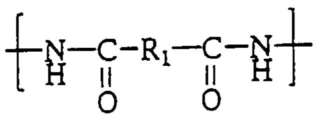

(3)

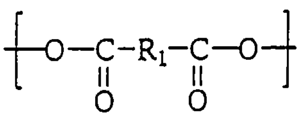

(4)

(where $R^1$, $R^2$ and $R^3$ represent bivalent substituted or non-substituted aliphatic group, alicyclic group or aromatic group).

2. A polyester amide copolymer characterized in that the copolymer has a repeated structure substantially represented by general formula (1) and that peak intensities α, β and γ based on diad sequence formulas (2), (3) and (4) which are observed in a $^{13}$C-NMR spectrum satisfy the following expression $70 \leq \alpha/(\alpha + \beta + \gamma) \times 100 \leq 100$ :

(1)

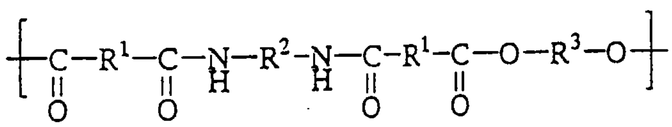

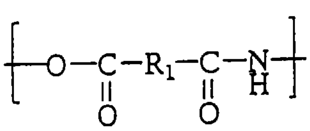

(2)

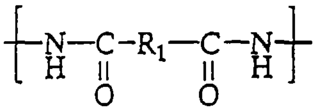

(3)

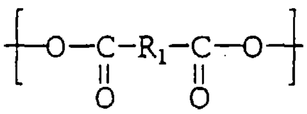

(4)

(where $R^1$, $R^2$ and $R^3$ represent bivalent substituted or non-substituted aliphatic group, alicyclic group or aromatic group).

3. A polyester amide copolymer according to the foregoing paragraph 1, characterized in that in general formulas (1), (2), (3) and (4), $R^1$, $R^2$ and $R^3$ have one or more groups selected from the following general formulas (5):

$-(CH_2)_l-$ , 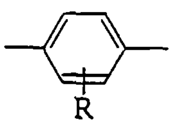 , 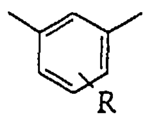 , 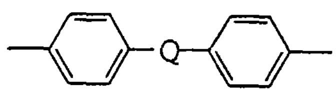 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

4. A polyester amide copolymer according to the foregoing paragraph 1, characterized in that general formulas (1), (2), (3) and (4) are represented by general formulas (6), (7), (8) and (9):

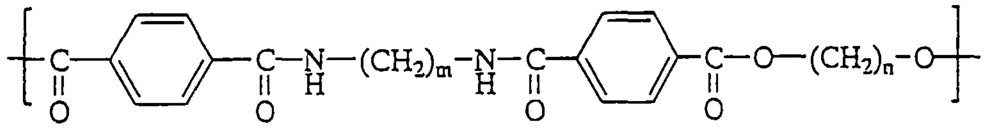 (6)

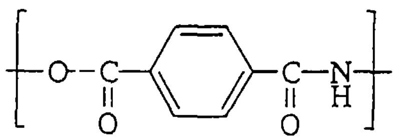 (7)

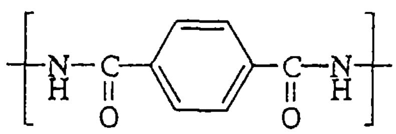 (8)

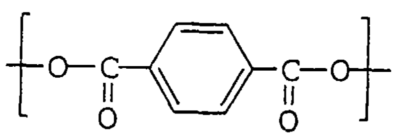 (9)

(where m and n represent integers equal to or greater than 2).

5. A polyester amide copolymer according to the foregoing paragraph 2, characterized in that in general formulas (1), (2), (3) and (4), $R^1$, $R^2$ and $R^3$ have one or more groups selected from general formulas (5):

$-(CH_2)_l-$ , 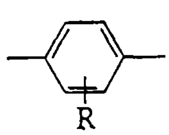 , 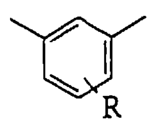 , 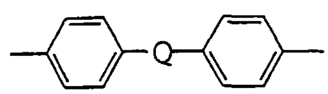 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

6. A polyester amide copolymer according to the foregoing paragraph 2, characterized in that general formulas (1), (2), (3) and (4) are represented by general formulas (6), (7), (8) and (9):

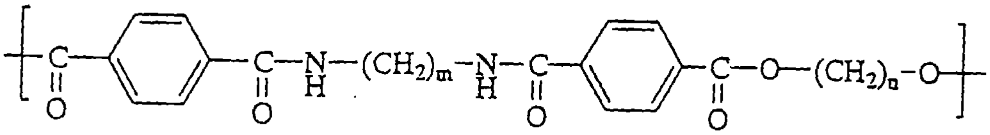

(6)

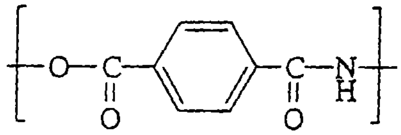

(7)

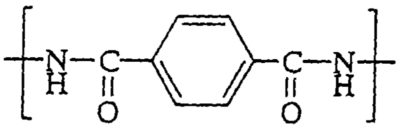

(8)

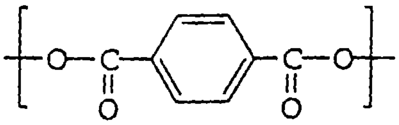

(9)

(where m and n in the above formulas represent integers equal to or greater than 2).

7. A polyester amide copolymer according to the foregoing paragraph 1, characterized in that a number average molecular weight of the polyester amide copolymer measured by gel permeation chromatography and then converted based on a polymethylmethacrylate is equal to or greater than 10 thousand.

8. A polyester amide copolymer according to the foregoing paragraph 3, characterized in that a number average molecular weight of the polyester amide copolymer measured by gel permeation chromatography and then converted based on a polymethylmethacrylate is equal to or greater than 10 thousand.

9. A polyester amide copolymer according to the foregoing paragraph 4, characterized in that a number average molecular weight of the polyester amide copolymer measured by gel permeation chromatography and then converted based on a polymethylmethacrylate is equal to or greater than 10 thousand.

10. A polyester amide copolymer according to the foregoing paragraph 2, characterized in that a number average molecular weight of the polyester amide copolymer measured by gel permeation chromatography and then converted based on a polymethylmethacrylate is equal to or greater than 10 thousand.

11. A polyester amide copolymer according to the foregoing paragraph 5, characterized in that a number average molecular weight of the polyester amide copolymer measured by gel permeation chromatography and then converted based on a polymethylmethacrylate is equal to or greater than 10 thousand.

12. A polyester amide copolymer according to the foregoing paragraph 6, characterized in that a number average molecular weight of the polyester amide copolymer measured by gel permeation chromatography and then converted based on a polymethylmethacrylate is equal to or greater than 10 thousand.

13. A polyester amide copolymer according to the foregoing paragraph 1, characterized in that a 5 wt.% reduction temperature is 370°C or higher.

14. A polyester amide copolymer according to the foregoing paragraph 3, characterized in that a 5 wt.% reduction temperature is 370°C or higher.

15. A polyester amide copolymer according to the foregoing paragraph 4, characterized in that a 5 wt.% reduction temperature is 370°C or higher.

16. A polyester amide copolymer according to the foregoing paragraph 2, characterized in that a 5 wt.% reduction temperature is 370°C or higher.

17. A polyester amide copolymer according to the foregoing paragraph 5, characterized in that a 5 wt.% reduction temperature is 370°C or higher.

18. A polyester amide copolymer according to the foregoing paragraph 6, characterized in that a 5 wt.% reduction temperature is 370°C or higher.

19. A polyester amide copolymer according to the foregoing paragraph 4, characterized in that $m = n = 6$, and a quantity of heat of melting is 50 J/g or greater.

20. A polyester amide copolymer according to the foregoing paragraph 6, characterized in that $m = n = 6$, and a quantity of heat of melting is 50 J/g or greater.

21. A polyester amide copolymer according to the foregoing paragraph 9, characterized in that $m = n = 6$, and a quantity of heat of melting is 50 J/g or greater.

22. A polyester amide copolymer according to the foregoing paragraph 12, characterized in that $m = n = 6$, and a quantity of heat of melting is 50 J/g or greater.

23. A polyester amide copolymer according to the foregoing paragraph 4, characterized in that m = 6, n = 4, and a quantity of heat of melting is 40 J/g or greater.

24. A polyester amide copolymer according to the foregoing paragraph 6, characterized in that m = 6, n = 4, and a quantity of heat of melting is 40 J/g or greater.

25. A polyester amide copolymer according to the foregoing paragraph 9, characterized in that m = 6, n = 4, and a quantity of heat of melting is 40 J/g or greater.

26. A polyester amide copolymer according to the foregoing paragraph 12, characterized in that m = 6, n = 4, and a quantity of heat of melting is 40 J/g or greater.

27. A polyester amide copolymer according to the foregoing paragraph 4, characterized in that m = 6, n = 2, and a quantity of heat of melting is 30 J/g or greater.

28. A polyester amide copolymer according to the foregoing paragraph 6, characterized in that m = 6, n = 2, and a quantity of heat of melting is 30 J/g or greater.

29. A polyester amide copolymer according to the foregoing paragraph 9, characterized in that m = 6, n = 2, and a quantity of heat of melting is 30 J/g or greater.

30. A polyester amide copolymer according to the foregoing paragraph 12, characterized in that m = 6, n = 2, and a quantity of heat of melting is 30 J/g or greater.

31. A method of producing a polyester amide copolymer characterized by heating an ester amide monomer represented by general formula (10) and a glycol represented by general formula (11) in the presence of a catalyst at a polymerization temperature of 150-350°C, and then further heating them at a pressure reduction degree of 0.01-100 mmHg at a polymerization temperature of 150°C-350°C:

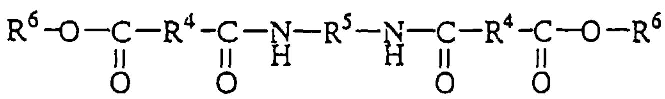 (10)

$$HO\text{-}R^7\text{-}OH \qquad (11)$$

(where $R^4$, $R^5$ and $R^7$ represent bivalent substituted or non-substituted aliphatic group, alicyclic group or aromatic group, and $R^6$ represents hydrogen or alkyl group of a carbon number of 1-5).

32. A method of producing a polyester amide copolymer according to the foregoing paragraph 31, characterized in that $R^4$, $R^5$ and $R^7$ in the ester amide monomer represented by general formula (10) and the glycol represented by general formula (11) are one or more groups selected from general formulas (5):

$-(CH_2)_l-$ , 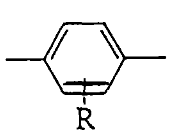 , 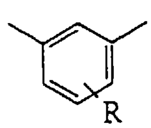 , 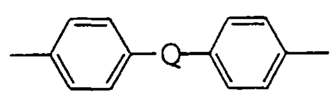 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group.

33. A method of producing a polyester amide copolymer according to the foregoing paragraph 31, characterized in that the ester amide monomer represented by general formula (10) and the glycol represented by general formula (11) are represented by general formulas (12) and (13):

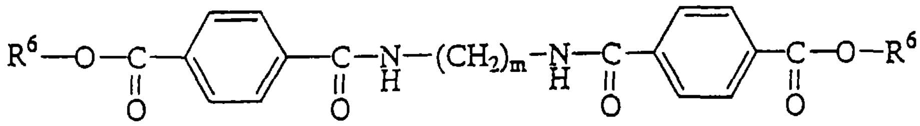 (12)

$$HO-(CH_2)_n-OH \qquad (13)$$

(where m and n represent integers equal to or greater than 2, and $R^6$ represents hydrogen or alkyl group of a carbon number of 1-5).

34. An ester amide monomer which is represented by general formula (10) and whose purity is 90 wt.% or higher:

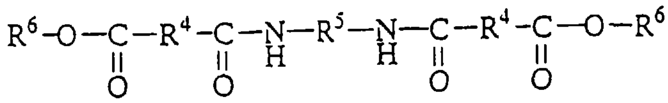 (10)

(where $R^4$ and $R^5$ represent bivalent substituted or non-substituted aliphatic group, alicyclic group or aromatic group, and $R^6$ represents hydrogen or alkyl group of a carbon number of 1-5).

35. An ester amide monomer according to the foregoing paragraph 34, characterized in that $R^4$ and $R^5$ in the ester amide monomer represented by general formula (10) are one or more groups selected from general formulas (5):

$-(CH_2)_l-$ , 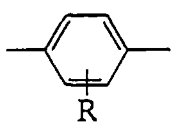 , 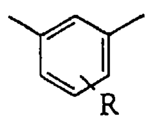 , 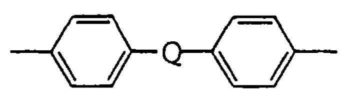 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

36. An ester amide monomer according to the foregoing paragraph 34, characterized in that the ester amide monomer represented by general formula (10) is represented by general formula (12):

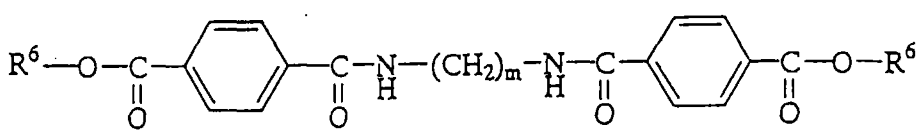 (12)

(where m represents an integer equal to or greater than 2, and $R^6$ represents hydrogen or alkyl group of a carbon number of 1-5).

37. An ester amide monomer which is represented by general formula (10) and whose purity is 95 wt.% or higher:

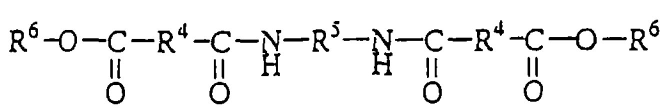 (10)

(where $R^4$ and $R^5$ represent bivalent substituted or non-substituted aliphatic group, alicyclic group or aromatic

group, and $R^6$ represents hydrogen or alkyl group of a carbon number of 1-5).

38. An ester amide monomer according to the foregoing paragraph 37, characterized in that $R^4$ and $R^5$ in the ester amide monomer represented by general formula (10) have one or more groups selected from general formulas (5):

$-(CH_2)_l-$ , 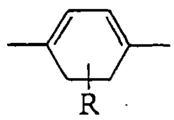 , 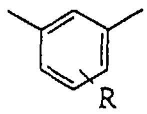 , 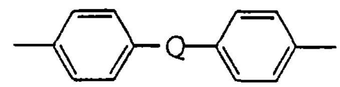 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

39. An ester amide monomer according to the foregoing paragraph 37, characterized in that the ester amide monomer represented by general formula (10) is represented by general formula (12):

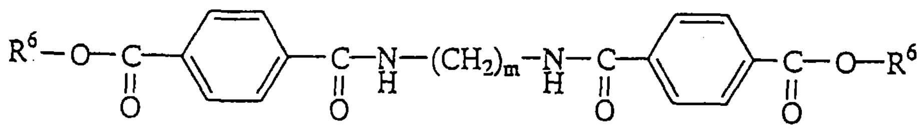 (12)

(where m represents an integer equal to or greater than 2, and $R^6$ represents hydrogen or alkyl group of a carbon number of 1-5).

40. A method of producing an ester amide monomer characterized by heating a diester compound represented by general formula (14) and a diamine compound represented by general formula (15) in the presence or absence of an organic solvent:

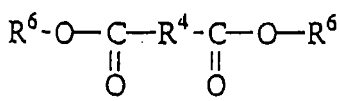 (14)

$$H_2N\text{-}R^5\text{-}NH_2 \quad (15)$$

(where $R^4$ and $R^5$ represent bivalent substituted or non-substituted aliphatic group, alicyclic group or aromatic group, and $R^6$ represents hydrogen or alkyl group of a carbon number of 1-5).

41. A method of producing an ester amide monomer according to the foregoing paragraph 40, characterized in that $R^4$ and $R^5$ in the diester compound represented by general formula (14) and the diamine compound represented by general formula (15) are one or more groups selected from general formulas (5):

$-(CH_2)_l-$ , 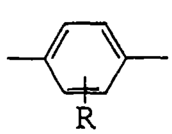 , 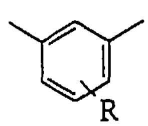 , 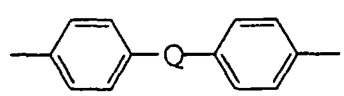 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

42. A method of producing an ester amide monomer according to the foregoing paragraph 40, characterized in that the diester compound represented by general formula (14) and the diamine compound represented by general formula (15) are a diester compound represented by general formula (16) and a diamine compound represented by

general formula (17):

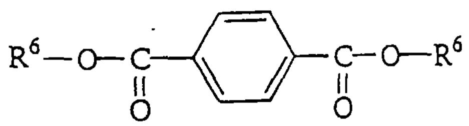

(16)

$$H_2N\text{-}(CH_2)_m\text{-}NH_2 \quad (17)$$

(where m represents an integer equal to or greater than 2, and $R^6$ represents alkyl group of a carbon number of 1-5).

43. A method of producing an ester amide monomer according to the foregoing paragraph 40, characterized in that the organic solvent has a characteristic that the organic solvent dissolves the diester compound represented by general formula (14) and the diamine compound represented by general formula (15) but does not dissolve the ester amide monomer.

44. A method of producing an ester amide monomer according to the foregoing paragraph 40, characterized in that the organic solvent has a characteristic that the organic solvent dissolves a diester compound represented by general formula (14) and a diamine compound represented by general formula (15) in which $R^4$ and $R^5$ are one or more groups selected from general formulas (5), but does not dissolve the ester amide monomer.

45. A method of producing an ester amide monomer according to the foregoing paragraph 40, characterized in that the organic solvent has a characteristic that the organic solvent dissolves a diester compound represented by general formula (16) and a diamine compound represented by general formula (17) but does not dissolve the ester amide monomer.

46. A method of producing an ester amide monomer according to the foregoing paragraph 40, characterized in that the diester compound represented by general formula (14) and the diamine compound represented by general formula (15) are heated in the absence of an organic solvent at a temperature equal to or higher than a melting point of the diester compound and lower than or equal to a melting point of the ester amide monomer.

47. A method of producing an ester amide monomer according to the foregoing paragraph 40, characterized in that there is a characteristic in that among the diester compound represented by general formula (14) and the diamine compound represented by general formula (15), a diester compound and a diamine compound in which $R^4$ and $R^5$ are one or more groups selected from general formulas (5) are dissolved, but the ester amide monomer is not dissolved.

48. A method of producing an ester amide monomer according to the foregoing paragraph 40, characterized in that a diester compound represented by general formula (16) and a diamine compound represented by general formula (17) are heated in the absence of an organic solvent at a temperature equal to or higher than a melting point of the diester compound and lower than or equal to a melting point of the ester amide monomer.

49. A method of producing an ester amide monomer according to the foregoing paragraph 40, characterized in that the mole ratio of the diamine compound to the diester is equal to or greater than 0.01-fold mole but less than or equal to 0.3-fold mole.

50. A method of producing an ester amide monomer according to the foregoing paragraph 40, characterized in that a species or a mixture of two or more species selected from monobutylhydroxy tinoxide, titanium tetrabutoxide, cesium fluoride, antimony fluoride, antimony oxides, metal alcoxides, ammonium salts, metal acetate salts, metal hydrides, organometallic compounds, alkaline inorganic compounds, and alkali metals, is used.

51. A method of producing a polyester amide copolymer according to the foregoing paragraph 31, characterized in that the ester amide monomer is an ester amide monomer obtained by heating a diester compound represented by general formula (14) and a diamine compound represented by general formula (15) in the presence or absence of an organic solvent.

52. A method of producing a polyester amide copolymer according to the foregoing paragraph 32, characterized in that the ester amide monomer is an ester amide monomer obtained by heating a diester compound represented by general formula (14) and a diamine compound represented by general formula (15) in which diester compound and diamine compound $R^4$ and $R^5$ are one or more groups selected from general formulas (5), in the presence or absence of an organic solvent.

53. A method of producing a polyester amide copolymer according to the foregoing paragraph 33, characterized in that the ester amide monomer is an ester amide monomer obtained by heating a diester compound represented by

general formula (16) and a diamine compound represented by general formula (17) in the presence or absence of an organic solvent.

54. A polyester amide resin composition which is obtained by further compounding a filler with 100 parts by weight of a polyester amide copolymer described in any one of the foregoing paragraphs 1-30, in an amount of 1-200 parts by weight relative to 100 parts by weight of the polyester amide copolymer.

55. A polyester amide resin composition which is obtained by further compounding an elastomer having a glass transition temperature of 20°C or lower with a polyester amide copolymer described in any one of the foregoing paragraphs 1-30, in an amount of 1-100 parts by weight relative to 100 parts by weight of the polyester amide copolymer.

56. A polyester amide resin composition according to the foregoing paragraph 55, characterized in that the elastomer having a glass transition temperature of 20°C or lower is an olefin-based elastomer.

57. A polyester amide resin composition which is obtained by further compounding a crystal nucleus agent with a polyester amide copolymer described in any one of the foregoing paragraphs 1-30 or a polyester amide resin composition described in the foregoing paragraph 54 or 55, in an amount of 0.01-20 parts by weight relative to 100 parts by weight of the polyester amide copolymer.

58. A polyester amide resin composition according to the foregoing paragraph 54, wherein the crystal nucleus agent is a species or a mixture of two or more species selected from talc, mica, kaolion, silica, clay, inorganic carboxylic acid salts, inorganic sulfonic acid salts, metal oxides, carbonic acid salts, organic carboxylic acid salts, and organic sulfonic acid salts.

59. A polyester amide resin composition which is obtained by further compounding a heat-resistant stabilizing agent with a polyester amide copolymer described in any one of the foregoing paragraphs 1-30 or a polyester amide resin composition described in the foregoing paragraph 54, 55 or 57, in an amount of 0.01-30 parts by weight relative to 100 parts by weight of the polyester amide copolymer.

60. A polyester amide resin composition which is obtained by further compounding a weathering-resistant stabilizing agent with a polyester amide copolymer described in any one of the foregoing paragraphs 1-30 or a polyester amide resin composition described in the foregoing paragraph 54, 55, 57 or 59, in an amount of 0.01-30 parts by weight relative to 100 parts by weight of the polyester amide copolymer.

61. A polyester amide resin composition which is obtained by further compounding a mold releasing agent with a polyester amide copolymer described in any one of the foregoing paragraphs 1-30 or a polyester amide resin composition described in the foregoing paragraph 54, 55, 57, 59 or 60, in an amount of 0.01-30 parts by weight relative to 100 parts by weight of the polyester amide copolymer.

62. A polyester amide resin composition which is obtained by further compounding a lubricant with a polyester amide copolymer described in any one of the foregoing paragraphs 1-30 or a polyester amide resin composition described in the foregoing paragraph 54, 55, 57, 59, 60 or 61, in an amount of 0.01-30 parts by weight relative to 100 parts by weight of the polyester amide copolymer.

63. A polyester amide resin composition which is obtained by further compounding an epoxy compound with a polyester amide copolymer described in any one of the foregoing paragraphs 1-30 or a polyester amide resin composition described in any one of the foregoing paragraphs 54, 55, 57 and 59-62, in an amount of 0.01-30 parts by weight relative to 100 parts by weight of the polyester amide copolymer.

64. A polyester amide resin composition which is obtained by further compounding a thermoplastic resin other than elastomers having glass transition temperatures of 20°C or lower, with a polyester amide copolymer described in any one of the foregoing paragraphs 1-30 or a polyester amide resin composition described in any one of the foregoing paragraphs 54, 55, 57 and 59-63, in an amount of 0.1-1000 parts by weight relative to 100 parts by weight of the polyester amide copolymer.

65. A form containing a polyester amide copolymer described in any one of the foregoing paragraphs 1-30 or a polyester amide resin composition described in any one of the foregoing paragraphs 54, 55, 57 and 59-64.

66. A fiber containing a polyester amide copolymer described in any one of the foregoing paragraphs 1-30 or a polyester amide resin composition described in any one of the foregoing paragraphs 54, 55, 57 and 59-64.

67. A film containing a polyester amide copolymer described in any one of the foregoing paragraphs 1-30 or a polyester amide resin composition described in any one of the foregoing paragraphs 54, 55, 57 and 59-64.

## BRIEF DESCRIPTION OF THE DRAWINGS

Fig. 1 is a flowchart of a solvent separation method for measurement of a purity of an ester amide monomer.

Fig. 2 is a diagram indicating a 1H-NMR spectrum of an ester amide monomer obtained in Example 1.

Fig. 3 is a diagram indicating an IR spectrum of an ester amide monomer obtained in Example 1.

Fig. 4 is a diagram indicating a 13C-NMR spectrum of a polyester amide copolymer obtained in Example 4.

Fig. 5 is a diagram indicating a 13C-NMR spectrum of a polyester amide copolymer obtained in Comparative

Example 2.

Fig. 6 is a diagram indicating a 13C-NMR spectrum of a polyester amide copolymer obtained in Example 6.

Fig. 7 is a diagram indicating a 13C-NMR spectrum of a polyester amide copolymer obtained in Comparative Example 3.

BEST MODES FOR CARRYING OUT THE INVENTION

The polyester amide copolymerization according to the present invention will be specifically described below. The polyester amide copolymer of the present invention comprises a repetition unit substantially represented by general formula (1) as described above:

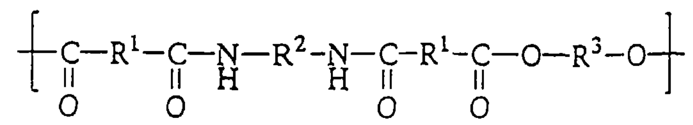 (1)

(where $R^1$, $R^2$ and $R^3$ represent bivalent substituted or non-substituted aliphatic group, alicyclic group or aromatic group).

In the structure of general formula (1), $R^1$, $R^2$ and $R^3$ represent bivalent substituted or non-substituted aliphatic group, alicyclic group or aromatic group, and may be the same or different from each other.

Preferred among the ester amid copolymers represented by general formula (1) are ester amid copolymers in which $R^1$, $R^2$ and $R^3$ have one or more groups selected from the following general formulas (5):

$-(CH_2)_l-$ , 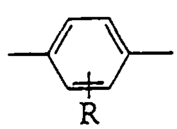 , 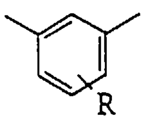 , 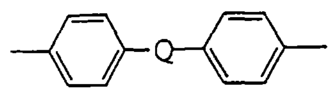 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

In the structure of general formula (5), l representing an integer equal to or greater than 2 is preferably 2-6 and, particularly preferably, 2, 4 or 6 and, even more preferably, 2 or 6. R represents hydrogen, halogen or a monovalent organic residue.

As specific examples of the monovalent organic residue, methyl group, ethyl group, n-propyl group, isopropyl group, n-butyl group, sec-butyl group, tert-butyl group, n-pentyl group, 2-pentyl group, 3-pentyl group, neopentyl group, phenyl group, xylyl group, cumenyl group, naphthyl group and the like may be cited. Methyl group, ethyl group and phenyl group are preferred, and methyl group and phenyl group are particularly preferred.

In the structure of general formula (5), Q represents a direct bond, O, S, $SO_2$, $C(CH_3)_2$, $CH_2$ or CHPh, Ph representing phenyl group.

Among the polyester amide copolymers represented by general formula (1), those represented by general formula (6) are particularly preferred in view of mechanical properties, crystallinity, heat resistance and forming-processability:

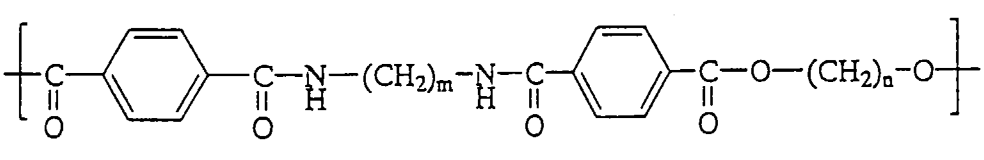 (6)

(where m and n represent integers equal to or greater than 2.

In the structure of general formula (6), m representing equal to or greater than 2 is preferably 2-6 and, particularly preferably, 2, 4 or 6 and, more preferably, 6, and n representing equal to or greater than 2 is preferably 2-6 and, particularly preferably, 2, 4 or 6 and, more preferably, 2.

The polyester amide copolymer of the present invention may contain repetition units represented by general formula (18), (19), in addition to the foregoing general formula (1):

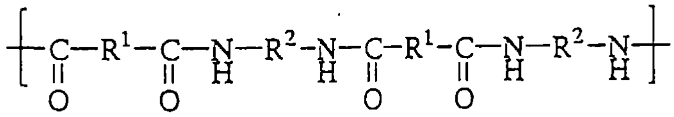 (18)

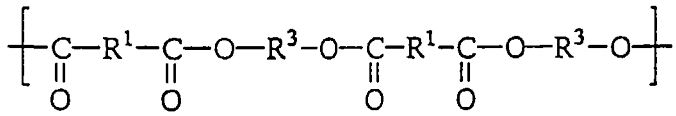 (19)

(where $R^1$, $R^2$ and $R^3$ represent bivalent substituted or non-substituted aliphatic group, alicyclic group or aromatic group).

In the structures of general formulas (18) and (19), $R^1$, $R^2$ and $R^3$ represent bivalent substituted or non-substituted aliphatic group, alicyclic group or aromatic group, and may be the same or different from each other.

In the structures of general formulas (18) and (19), $R^1$, $R^2$ and $R^3$ have one or more groups selected from the following general formulas (5):

$-(CH_2)_l-$ , 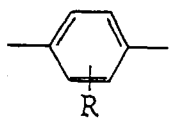 , 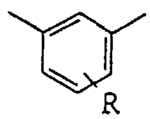 , 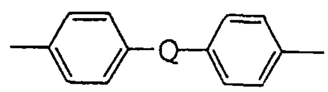 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

In the structure of general formula (5), l representing an integer equal to or greater than 2 is preferably 2-6 and, particularly preferably, 2, 4 or 6 and, even more preferably, 2 or 6. R represents hydrogen, halogen or a monovalent organic residue.

As specific examples of the monovalent organic residue, methyl group, ethyl group, n-propyl group, isopropyl group, n-butyl group, sec-butyl group, tert-butyl group, n-pentyl group, 2-pentyl group, 3-pentyl group, neopentyl group, phenyl group, xylyl group, cumenyl group, naphthyl group and the like may be cited. Methyl group, ethyl group and phenyl group are preferred, and methyl group and phenyl group are particularly preferred.

In the structure of general formula (5), Q represents a direct bond, O, S, $SO_2$, $C(CH_3)_2$, $CH_2$ or CHPh, Ph representing phenyl group.

Depending on the structure of the polyester amide copolymer, the repetition units represented by the foregoing general formulas (18) and (19) have repetition units represented by the following general formulas (20) and (21):

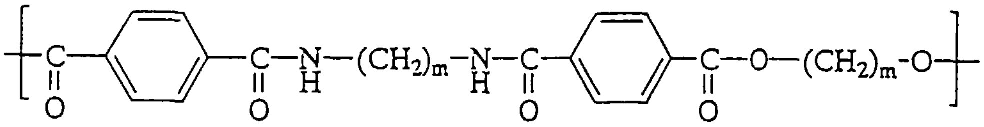 (20)

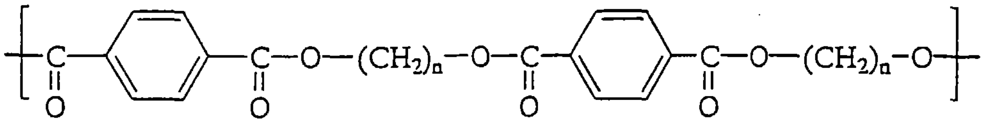 (21)

(where m and n represent integers equal to or greater than 2).

The mole percents of the repetition units represented by the foregoing general formulas (1), (18) and (19) can be calculated from the diad sequences represented by the diad sequences (2), (3) and (4).

That is, the calculation is possible based on the mole percents of the diad sequences represented by (2), (3) and (4):

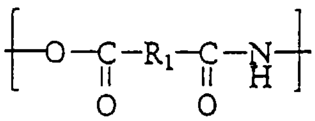 (2)

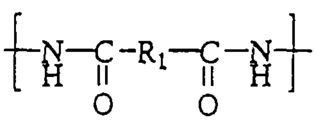 (3)

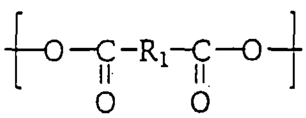 (4)

(where $R^1$ represents bivalent substituted or non-substituted aliphatic group, alicyclic group or aromatic group).

In the structures of the foregoing general formulas (2), (3) and (4), $R^1$ represents bivalent substituted or non-substituted aliphatic group, alicyclic group or aromatic group, and may be the same or different from each other.

In the structures of the foregoing general formulas (2), (3) and (4), $R^1$ has one or more groups selected from the following general formulas (5), depending on the structure of the polyester amide copolymer:

$-(CH_2)_l-$ , 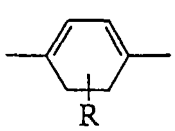 , 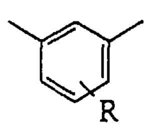 , 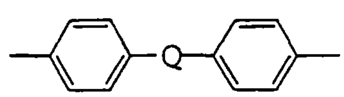 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

In the structure of general formula (5), l representing an integer equal to or greater than 2 is preferably 2-6 and, particularly preferably, 2, 4 or 6 and, even more preferably, 2 or 6. R represents hydrogen, halogen or a monovalent organic residue.

As specific examples of the monovalent organic residue, methyl group, ethyl group, n-propyl group, isopropyl group, n-butyl group, sec-butyl group, tert-butyl group, n-pentyl group, 2-pentyl group, 3-pentyl group, neopentyl group, phenyl group, xylyl group, cumenyl group, naphthyl group and the like may be cited. Methyl group, ethyl group and phenyl group are preferred, and methyl group and phenyl group are particularly preferred.

In the structure of general formulas (2), (3) and (4), Q represents a direct bond, O, S, $SO_2$, $C(CH_3)_2$, $CH_2$ or CHPh, Ph representing phenyl group.

The diad sequences represented by the foregoing general formulas (2), (3) and (4) have diad sequences represented by the following general formulas (7), (8) and (9), depending on the structure of the polyester amide copolymer:

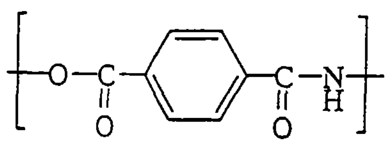

(7)

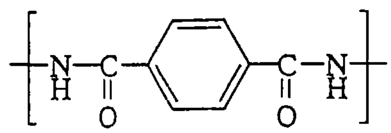

(8)

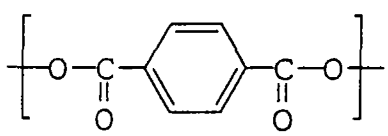

(9)

In the polyester amide copolymer of the present invention, the mole percents (X) of the diad sequence in the polyester amide copolymer is $70 \leq X \leq 100$ and, preferably, $80 \leq X \leq 100$ and, more preferably, $90 \leq X \leq 100$.

As the value X increases, the mole % of the repetition unit represented by general formula (1) in the polyester amide copolymer becomes higher and the mole % of the repetition units represented by general formulas (18), (19) becomes lower, which means the alternation in the polyester amide copolymer is high. Polyester amide copolymers having high alternations exhibit preferable characteristics in crystallinity, heat resistance, mechanical properties and forming-processability.

Depending on the structure of the polyester amide copolymer, the diad sequence represented by the foregoing formula (2) may be a diad sequence having one or more groups selected from the foregoing general formulas (5) and, furthermore, the diad sequence represented by general formula (2) may have a diad sequence represented by the foregoing general formula (7).

The method of measuring mole percents of the diad sequences of (2), (3) and (4) is not particularly limited. It may be calculated from the peak intensities $\alpha$, $\beta$ and $\gamma$ based on diad sequence formulas (2), (3) and (4) that are observed in a 13C-NMR spectrum.

The peak intensities of $\alpha$, $\beta$ and $\gamma$ of the polyester amide copolymer of the present invention based on (2), (3) and (4) satisfy the following general expression: $70 \leq \alpha/(\alpha + \beta + \gamma) \times 100 \leq 100$, and preferably, $80 \leq \alpha/(\alpha + \beta + \gamma) \times 100 \leq 100$, and more preferably, $90 \leq \alpha/(\alpha + \beta + \gamma) \times 100 \leq 100$.

A higher proportion indicates a higher alternation. Polyester amide copolymers having high alternations exhibit preferable characteristics in crystallinity, heat resistance, mechanical properties and forming-processability.

The method of calculating $\alpha$, $\beta$ and $\gamma$, and the relationship among the mole percents (X, Y and Z) of the diad sequences (2), (3) and (4) in the ester amide copolymer represented by general formula (1) will be described.

As an example, diad sequences (7), (8) and (9) in the polyester amide copolymer represented by the foregoing general formula (6) will be described in detail.

As for the diad sequences represented by the foregoing general formulas (7), (8) and (9), α, β and γ can be calculated from signal intensities deriving from aromatic carbons linked to carbonyl groups among the aromatic carbons in general formulas (7), (8) and (9). That is, in a 13C-NMR spectrum, signals deriving from the diad sequences represented by general formula (7) are observed at 138 ppm and 133 ppm if measurement has been performed in deuterated hexafluoroisopropanol. The sum of the intensities of the two signals is defined as α.

A signal deriving from the diad sequence represented by general formula (8) is observed at 137 ppm. The intensity of this signal is defined as β.

A signal deriving from the diad sequence represented by general formula (9) is observed at 134 ppm. The intensity of this signal is defined as γ.

Since in any of the cases, they derive from the aromatic carbons linked to carbonyl groups among the aromatic carbons, it can be considered that their relaxation times are the same, and that the signal intensity has a correlation with the mole number of the aromatic carbons linked to carbonyl groups. Therefore, α, β and γ represent the mole percents (X, Y and Z) of the diad sequences represented by general formulas (7), (8) and (9). That is, the mole percents (X, Y and Z) of the diad sequences represented by general formulas (7), (8) and (9) can be calculated as $X = \alpha/(\alpha + \beta + \gamma) \times 100$, $Y = \beta/(\alpha + \beta + \gamma) \times 100$, and $Z = \gamma/(\alpha + \beta + \gamma) \times 100$.

The number average molecular weight of the polyester amide copolymer of the present invention determined by performing measurement by GPC (gel permeation chromatography) and then conversion based on standard polymethylmethacrylate is 10 thousand or greater, and preferably 15 thousand or greater, and more preferably 20 thousand or greater. Polyester amide copolymer having a number average molecular weight less than 10 thousand is unpreferable since they are inferior in mechanical properties and processability. There is no particular upper limit for the number average molecular weight of the polyester amide copolymer. However, it is preferred that the number average molecular weight be within a range such that molten molding or forming is possible.

The temperature at which the polyester amide copolymer of the invention is reduced by 5 wt.% is 370°C or higher and, preferably, 380°C or higher, and more preferably, 390°C or higher.

The 5 wt.% reduction temperature refers to a temperature occurring when a weight reduction reaches 5% in a 100°C-850°C temperature increase at a rate of 20°C/min in a nitrogen atmosphere using a sample amount of 10 mg and a TGA (thermogravimetric analyzer).

Among polyester amide copolymers according to the present invention, a polyester amide copolymer represented by the foregoing general formula (6) wherein m is 6, preferably has a differential scanning calorimeter (DSC)-measured heat of melting (ΔH) that is greater than a certain value, in view of heat resistance, crystallinity, mechanical properties and forming-processability. The heat of melting varies depending on the value of n.

If $m = n = 6$, the heat of melting is preferably 50 J/g or greater, and more preferably 55 J/g, in view of heat resistance, crystallinity, mechanical properties and forming-processability.

If $m = 6$ and $n = 4$, the heat of melting is preferably 40 J/g or greater, and more preferably 45 J/g, in view of heat resistance, crystallinity, mechanical properties and forming-processability.

If $m = 6$ and $n = 2$, the heat of melting is preferably 30 J/g or greater, and more preferably 35 J/g, in view of heat resistance, crystallinity, mechanical properties and forming-processability.

The heat of melting herein refers to a quantity of heat of an endothermic peak determined by a peak of an endothermic peak temperature (Tm2) observed in measurement using a differential calorimeter. After observation of an endothermic peak temperature (Tm1) that is to be observed in measurement of a polymer in a condition where the temperature is increased from 100°C at a rate of 20°C/min, the polymer is kept at a temperature of Tm1 + 20°C for 10 minutes, and then cooled to -100°C under a temperature decrease condition of 20°C/min and kept at the temperature for 15 minutes. Then measurement is performed again under a temperature increasing condition of 20°C/min, during which the endothermic peak temperature (Tm2) is observed.

The polyester amide copolymer as described above can be produced by a method described below. That is, the polyester amide copolymer is produced by heating an ester amide monomer represented by general formula (10) and a glycol represented by general formula (11) in the presence of a catalyst at a polymerisation temperature of 150°C-350°C and then heating them at a pressure reduction degree of 0.01-100 mmHg at a polymerization temperature of 150°C-350°C:

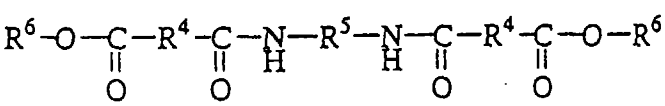

(10)

$$HO\text{-}R^7\text{-}OH \qquad (11)$$

(where $R^4$, $R^5$ and $R^7$ represent bivalent substituted or non-substituted aliphatic group, alicyclic group or aromatic group, and $R^6$ represents hydrogen or alkyl group of a carbon number of 1-5).

The ester amide monomer and the glycol used according to the present invention are represented by the foregoing general formula (10) and general formula (11).

In the structures of the foregoing general formulas (10) and (11), $R^4$, $R^5$ and $R^7$ represent bivalent substituted or non-substituted aliphatic group, alicyclic group or aromatic group, and may be the same or different from each other. $R^6$ represents hydrogen or alkyl group of a carbon number of 1-5.

As specific examples of the alkyl group of a carbon number of 1-5, methyl group, ethyl group, n-propyl group, isopropyl group, n-butyl group, sec-butyl group, tert-butyl group, n-pentyl group, 2-pentyl group, 3-pentyl group, neopentyl group and the like may be cited. Methyl group and ethyl group are preferred, and methyl group is particularly preferred.

Among the ester amide monomers represented by the foregoing general formula (10) and the glycols represented by the foregoing general formula (11), those in which $R^4$, $R^5$ and $R^7$ are one or more groups selected from the structures of general formulas (5) may preferably be used:

$-(CH_2)_l-$ , 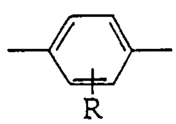 , 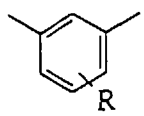 , 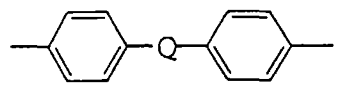 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

In the structure of general formula (5), l representing an integer equal to or greater than 2 is preferably 2-6 and, particularly preferably, 2, 4 or 6 and, even more preferably, 2 or 6. R represents hydrogen, halogen or a monovalent organic residue.

As specific examples of the monovalent organic residue, methyl group, ethyl group, n-propyl group, isopropyl group, n-butyl group, sec-butyl group, tert-butyl group, n-pentyl group, 2-pentyl group, 3-pentyl group, neopentyl group, phenyl group, xylyl group, cumenyl group, naphthyl group and the like may be cited. Methyl group, ethyl group and phenyl group are preferred, and methyl group and phenyl group are particularly preferred.

In the structure of general formula (5), Q represents a direct bond, O, S, $SO_2$, $C(CH_3)_2$, $CH_2$ or CHPh, Ph representing phenyl group.

Among the ester amide monomers represented by general formula (10), those represented by general formula (12) are preferred in view of the mechanical properties, crystallinity, heat resistance and forming-processability of the resultant polyester amide copolymer:

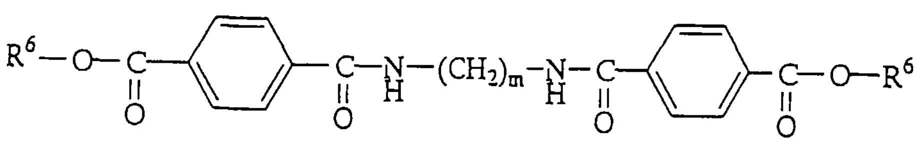 (12)

(where m represents an integer equal to or greater than 2, and $R^6$ represents hydrogen or alkyl group of a carbon number of 1-5).

In the structure of general formula (12), m representing an integer equal to or greater than 2 is preferably 2-6 and, particularly preferably, 4 or 6 and, even more preferably, 6. $R^6$ represents hydrogen or hydrogen or alkyl group of a carbon number of 1-5. As specific examples of the alkyl group of a carbon number of 1-5, methyl group, ethyl group, n-propyl group, isopropyl group, n-butyl group, sec-butyl group, tert-butyl group, n-pentyl group, 2-pentyl group, 3-pentyl group, neopentyl group and the like may be cited. $R^6$ is preferably hydrogen, methyl group or ethyl group, and particularly preferably, methyl or hydrogen.

Among the glycols represented by the foregoing general formula (11), those represented by general formula (13) are preferred in view of the mechanical properties, crystallinity, heat resistance and forming-processability of the resultant polyester amide copolymer:

$$HO\text{-}(CH_2)_n\text{-}OH \quad (13)$$

(where n represents an integer equal to or greater than 2).

In the structure of the foregoing general formula (13), n representing an integer equal to or greater than 2 is preferably 2-6 and, particularly preferably, 2, 4 or 6 and, more preferably, 2.

According to the present invention, the mole ratio of the glycol to the ester amide monomer is normally equal to or greater than 1-fold mole but less than or equal to 20-fold mole, and preferably, equal to or greater than 1-fold mole but less than or equal to 15-fold mole, and more preferably, equal to or greater than 1-fold mole but less than or equal to 10-fold mole, and particularly preferably, equal to or greater than 2-fold mole but less than or equal to 5-fold mole.

In polymerization of the polyester amide copolymer according to the present invention, it is preferred to first heat an ester amide monomer represented by the foregoing general formula (10) and a glycol represented by the foregoing general formula (11) in the presence of a catalyst at a polymerisation temperature of 150°C-350°C.

The polymerization time in this stage is equal to or longer than 0 hour but shorter than or equal to 10 hours, and preferably equal to or longer than 0.5 hour but shorter than or equal to 5 hours, and more preferably, equal to or longer than 1 hour but shorter than or equal to 4 hours.

Furthermore, according to the present invention, it is preferred that after heating at a polymerization temperature of 150°C-350°C, further heating at a pressure reduction degree 0.01-100 mmHg at a polymerization temperature of 150°C-350°C be performed.

The pressure reduction degree in this stage is normally equal to or higher than 0.01 mmHg but lower than or equal to 100 mmHg, and preferably, equal to or higher than 0.1 mmHg but lower than or equal to 50 mmHg, and more preferably, equal to or higher than 0.1 mmHg but lower than or equal to 10 mmHg, in view of the molecular weight of the resultant polymer.

The polymerization temperature in this stage is preferably equal to or higher than 150°C but lower than or equal to 350°C, and more preferably, equal to or higher than 200°C but lower than or equal to 300°C.

As for a polymerization catalyst used in the production of a polyester amide copolymer according to the present invention, a catalyst known as a polymerization catalyst for normal polyester may be used. For example, a species or a mixture of two or more species selected from monobutylhydroxy tinoxide, titanium tetrabutoxide, cesium fluoride, antimony fluoride, antimony trioxide, antimony pentaoxide, manganese acetate, cobalt acetate, potassium acetate and lithium acetate may be used. The catalyst is preferably a species or a mixture of two or more species selected from monobutylhydroxy tinoxide, titanium tetrabutoxide, antimony trioxide, antimony pentaoxide and manganese acetate, and more preferably, a species or a mixture of two or more species selected from titanium tetrabutoxide, antimony trioxide and manganese acetate.

There is no particular limit for the amount of a polymerization catalyst used. Normally, the percentage by weight of the polymerization catalyst relative to the total weight of the ester amide monomer and the glycol is preferably 0.001-1.0 wt.% and, more preferably, 0.01-0.5 wt.%.

The polymerization catalyst used in the present invention may be used in an intact form or in a diluted form using a glycol to be used in the polymerization.

The polymerization time remarkably varies depending on the amount of catalyst, the reaction temperature, the pressure reduction degree and the like. The polymerization time is normally 0.1-20 hours, and preferably, 0.2-10 hours, and more preferably, 0.5-8 hours.

The polyester amide copolymer thus obtained may be used immediately after being ejected from the polymerization apparatus, or may be used after it is ejected and then washed with a solvent or purified by re-precipitation. It is also possible to solid phase-polymerize the polyester amide copolymer obtained by the polymerization, at a temperature equal to or higher than the glass transition temperature but lower than or equal to the melting point of the polyester amide copolymer.

Next, the method of producing an ester amide monomer represented by the foregoing general formula (10) and, in particular, a high-purity ester amide monomer, will be specifically described.

The method produces an ester amide monomer represented by the foregoing general formula (10) and having a purity of 90 wt.% or higher in most cases. In preferred cases, an ester amide monomer having a purity of 92 wt.% or higher can be produced, and in more preferred cases, an ester amide monomer having a purity of 95 wt.% or higher can be produced. A polyester amide copolymer produced by using such a high-purity ester amide monomer is preferable in view of molecular weight, heat stability, heat resistance, dwell stability, non-tinting characteristic of polymer, crystallinity and forming-processability.

The method of measuring the purity of the ester amide monomer is not particularly limited. The measurement may be performed by a normal known analysis method such as liquid chromatography, gas chromatography, 1H-NMR and the like, and further by a solvent separation method wherein the ester monomer is separated and recovered.

As for the solvent separation method, measurement can normally be performed by the following method. That is, the ester amide monomer represented by the foregoing general formula (10) contains not only the compounds represented by the foregoing general formulas (14) and (15) but also compounds represented by the following general formulas (22), (23) and (24) and the like as impurities:

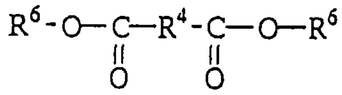

(14)

$$H_2N\text{-}R^5\text{-}NH_2 \quad (15)$$

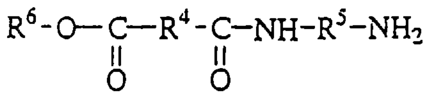

(22)

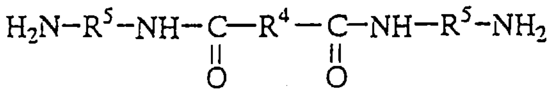

(23)

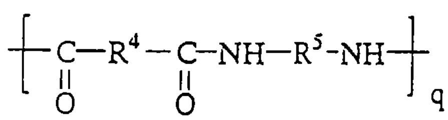

(24)

(where $R^4$ and $R^5$ represent bivalent substituted or non-substituted aliphatic group, alicyclic group or aromatic group, and $R^6$ represents hydrogen or alkyl group of a carbon number of 1-5, and q represents an integer equal to or greater than 2).

In the structures of the foregoing general formulas (14), (15), (22), (23) and (24), $R^4$ and $R^5$ represent bivalent substituted or non-substituted aliphatic group, alicyclic group or aromatic group, and may be the same or different from each other.

$R^6$ represents hydrogen or alkyl group of a carbon number of 1-5.

As specific examples of the alkyl group of a carbon number of 1-5, methyl group, ethyl group, n-propyl group, isopropyl group, n-butyl group, sec-butyl group, tert-butyl group, n-pentyl group, 2-pentyl group, 3-pentyl group, neopentyl group and the like may be cited. Methyl group and ethyl group are preferred, and methyl group is particularly preferred.

In the foregoing general formula, q represents an integer equal to or greater than 2.

In the structures of the foregoing general formula(14), (15), (22), (23) and (24), $R^4$ and $R^5$ have one or more groups selected from the structures of general formulas (5), depending on the structure of the corresponding ester amide monomer:

$-(CH_2)_l-$ , 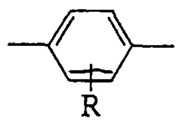 , 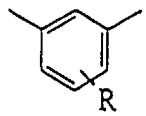 , 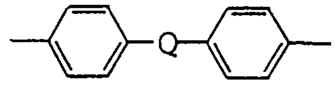 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

In the foregoing general formula (5), l representing an integer equal to or greater than 2 is preferably 2-6 and, particularly preferably, 2, 4 or 6 and, even more preferably, 2 or 6. R represents hydrogen, halogen or a monovalent organic residue.

As specific examples of the monovalent organic residue, methyl group, ethyl group, n-propyl group, isopropyl group, n-butyl group, sec-butyl group, tert-butyl group, n-pentyl group, 2-pentyl group, 3-pentyl group, neopentyl group, phenyl group, xylyl group, cumenyl group, naphthyl group and the like may be cited. Methyl group, ethyl group and phenyl group are preferred, and methyl group and phenyl group are particularly preferred.

In general formula (5), Q represents a direct bond, O, S, $SO_2$, $C(CH_3)_2$, $CH_2$ or CHPh, Ph representing phenyl group.

The compounds represented by the foregoing general formulas (14), (15), (22), (23) and (24) have structures represented by the following general formulas (16), (17), (25), (26) and (27), depending on the structure of the corresponding polyester amide copolymer:

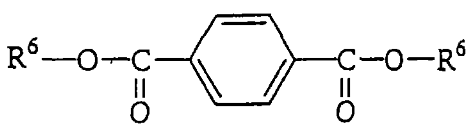 (16)

$$H_2N\text{-}(CH_2)_m\text{-}NH_2 \qquad (17)$$

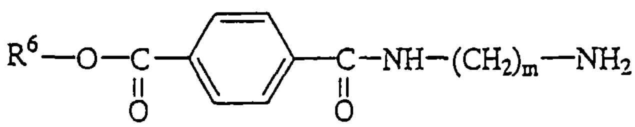 (25)

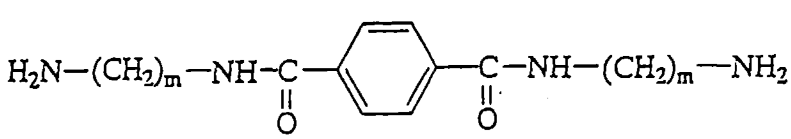 (26)

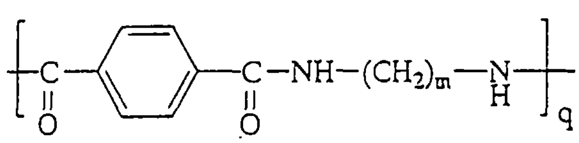 (27)

(where m represents an integer equal to or greater than 2, and $R^6$ represents alkyl group of a carbon number of 1-5, and q represents an integer equal to or greater than 2).

Utilizing the difference in solubility in an solvent between the ester amide monomer represented by the foregoing general formula (10) and the compounds represented by the foregoing general formulas (14), (15), (22), (23) and (24) and the like or the difference in solubility in an solvent between the ester amide monomer represented by general formula (12) and the compounds represented by the foregoing general formulas (16), (17), (25), (26) and (27) and the like, the ester amide monomer alone can be separated using the solvent. The purity of the ester amide monomer can be measured from the weight of the separated ester amide monomer.

An example of the purity measurement of an ester amide monomer represented by the foregoing general formula (12) using a solvent separation method is illustrated in the flowchart of Fig. 1.

A sample of 500.0 g is accurately measured out, and a 1L of DMF is added thereto, and the mixture is stirred at 20°C for 5 hours. A DMF insoluble fraction is recovered by filtration or centrifugation. The recovery is dissolved in 1L of hexafluoroisopropanol (HFIP), and the mixture is stirred at 20°C for 5 hours. Then, an HFIP insoluble fraction is removed by filtration or centrifugation. After the HFIP solution is evaporated, the weight (W) of the HFIP soluble fraction is measured.

The HFIP soluble fraction is an ester amide monomer represented by the foregoing general formula (12), and the DMF soluble fraction is an impurity component that includes, for example, not only the compounds represented by the foregoing general formulas (16) and (17) but also the compounds represented by the foregoing general formulas (25), (26) and (27) and the like.

By the method described above, the purity of the polyester amide monomer can be calculated as $W/500\times100$ (wt.%).

A high-purity ester amide monomer according to the present invention as described above can be produced from a diester compound represented by general formula (14) and a diamine compound represented by general formula (15):

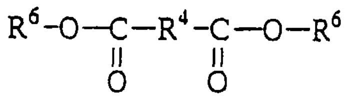 (14)

$$H_2N\text{-}R^5\text{-}NH_2 \qquad (15)$$

(where $R^4$ and $R^5$ represent bivalent substituted or non-substituted aliphatic group, alicyclic group or aromatic group, and $R^6$ represents hydrogen or alkyl group of a carbon number of 1-5).

According to the present invention, an ester amide monomer represented by general formula (10) can be produced by heating a diester compound represented by the foregoing general formula (14) and a diamine compound represented by the foregoing general formula (15) in an organic solvent or without using an organic solvent.

In the structural formulas of the diester compound represented by the foregoing general formula (14) and the diamine compound represented by the foregoing general formula (15), $R^4$ and $R^5$ represent bivalent substituted or non-substituted aliphatic group, alicyclic group or aromatic group, and may be the same or different from each other. $R^6$ represents hydrogen or alkyl group of a carbon number of 1-5.

As specific examples of the alkyl group of a carbon number of 1-5, methyl group, ethyl group, n-propyl group, isopropyl group, n-butyl group, sec-butyl group, tert-butyl group, n-pentyl group, 2-pentyl group, 3-pentyl group, neopentyl group and the like may be cited. Methyl group and ethyl group are preferred, and methyl group is particularly preferred.

Among the structures of the diester compound represented by the foregoing general formula (14) and the diamine compound represented by the foregoing general formula (15), those wherein $R^4$ and $R^5$ have one or more groups selected from the structures of general formulas (5) may preferably be used:

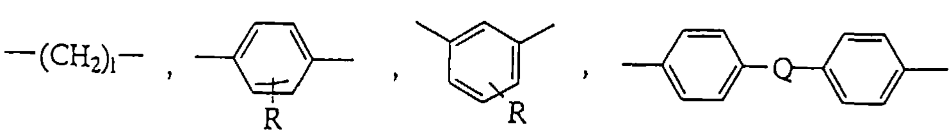 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

In general formula (5), l representing an integer equal to or greater than 2 is preferably 2-6 and, particularly preferably, 2, 4 or 6 and, even more preferably, 2 or 6. R represents hydrogen, halogen or a monovalent organic residue.

As specific examples of the monovalent organic residue, methyl group, ethyl group, n-propyl group, isopropyl group, n-butyl group, sec-butyl group, tert-butyl group, n-pentyl group, 2-pentyl group, 3-pentyl group, neopentyl group, phenyl group, xylyl group, cumenyl group, naphthyl group and the like may be cited. Methyl group, ethyl group and phenyl group are preferred, and methyl group and phenyl group are particularly preferred.

In general formula (5), Q represents a direct bond, O, S, $SO_2$, $C(CH_3)_2$, $CH_2$ or CHPh, Ph representing phenyl group.

Among the diester compounds represented by the foregoing general formula (14) and the diamine compounds represented by the foregoing general formula (15), diester compounds represented by general formula (16) and diamine compounds represented by general formula (17) may be particularly preferably used:

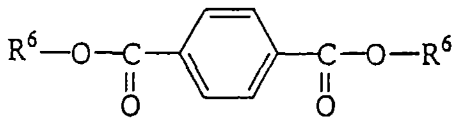

(16)

$$H_2N\text{-}(CH_2)_m\text{-}NH_2 \qquad (17)$$

(where m represents an integer equal to or greater than 2, and $R^6$ represents alkyl group of a carbon number of 1-5).

In the foregoing general formula (16), $R^6$ represents alkyl group of a carbon number of 1-5. As specific examples of the alkyl group of a carbon number of 1-5 herein, methyl group, ethyl group, n-propyl group, isopropyl group, n-butyl group, sec-butyl group, tert-butyl group, n-pentyl group, 2-pentyl group, 3-pentyl group, neopentyl group and the like may be cited. $R^6$ is preferably hydrogen, methyl group or ethyl group, and particularly preferably, methyl or hydrogen.

In the foregoing general formula (17), m representing an integer equal to or greater than 2 is preferably 2-6 and, particularly preferably, 4 or 6 and, even more preferably, 6.

The mole ratio of the diamine compound to the diester compound according to the present invention is preferably equal to or greater than 0.01-hold mole but less than or equal to 0.3-fold mole and, more preferably, equal to or greater than 0.05-fold mole but less than or equal to 0.2-fold mole, in view of the purity of the resultant ester amide monomer.

Furthermore, it is preferred to use a catalyst in the reaction for acceleration of the reaction. As such a catalyst, a known catalyst may normally be used. For example, a species or a mixture of two ore more species selected from monobutylhydroxy tinoxide, titanium tetrabutoxide, cesium fluoride, antimony fluoride, antimony oxides, metal alcoxides, ammonium salts, metal acetate salts, metal hydrides, organometallic compounds, and alkaline inorganic compounds, may be used. The catalyst is preferably a species or a mixture of two ore more species selected from monobutylhydroxy tinoxide, titanium tetrabutoxide, antimony oxides, metal alcoxides, metal acetate salts and alkaline inorganic compounds, and more preferably, a species or a mixture of two or more species selected from titanium tetrabutoxide, metal alcoxides, metal acetate salts and alkaline inorganic compounds.

As specific examples of the metal alcoxides, lithium methoxide, sodium methoxide, potassium methoxide, lithium ethoxide, sodium ethoxide, potassium ethoxide, lithium butoxide, sodium butoxide, potassium butoxide and the like may be cited. The metal alcoxides are preferably lithium methoxide, sodium methoxide, potassium methoxide, lithium ethoxide, sodium ethoxide and potassium ethoxide, and more preferably, lithium methoxide, sodium methoxide and potassium methoxide.

As specific examples of the ammonium salts, ammonium chloride, ammonium bromide, ammonium sulfate, ammonium phosphate, ammonium nitrate and the like may be cited. The ammonium salts are preferably ammonium chloride, ammonium bromide, ammonium sulfate and ammonium phosphate, and more preferably, ammonium chloride and ammonium bromide.

As specific examples or the metal acetate salts, lithium acetate, sodium acetate, potassium acetate, cobalt acetate, manganese acetate, aluminum acetate and the like may be cited. The metal acetate salts are preferably lithium acetate, sodium acetate, cobalt acetate and manganese acetate, and more preferably, lithium acetate and manganese acetate.

As specific examples of the metal hydrides, sodium hydride, potassium hydride, lithium hydride and the like may be cited. The metal hydrides are preferably sodium hydride and potassium hydride, and more preferably, sodium hydride.

The organometallic compounds are not particularly limited as long as they have an ionic bond or covalent bond of

carbon-metal. As specific examples of the organometallic compounds, organic aluminum compounds such as triethyl aluminum, trimethyl aluminum, triisobutyl aluminum and the like, alkyl lithium such as n-butyl lithium, t-butyl lithium and the like, and Grignard reagents such as methyl magnesium chloride, methyl magnesium bromide, ethyl magnesium chloride, ethyl magnesium bromide, phenyl magnesium chloride, phenyl magnesium bromide and the like may be cited.

As specific examples of the alkaline inorganic compounds, lithium hydroxide, sodium hydroxide, potassium hydroxide, magnesium hydroxide, calcium hydroxide, barium hydroxide and the like, may be cited. The alkaline inorganic compounds are preferably lithium hydroxide, sodium hydroxide and potassium hydroxide, and more preferably, sodium hydroxide.

The amount of a reaction catalyst used is not particularly limited. Normally, the percentage by weight of the catalyst relative to the total weight of the diester compound and the diamine compound is preferably 0.001-1.0 wt.% and, more preferably, 0.01-0.5 wt.%.

If the reaction is carried out in a solvent, an organic solvent used is preferably a solvent that dissolves the diester and diamine compounds but does not dissolve the ester amide monomer, in view of the purity of the resultant ester amide monomer. The organic solvent varies depending on the structures of the diester compound and the diamine compound used and the structure of the resultant ester amide monomer. However, the organic solvent is preferably a species or a mixture of two or more species selected from methanol, ethanol, pentane, hexane, heptane, benzene, toluene, xylene, cyclohexane, tetralin, decalin, petroleum, kerosene, chlorobenzene, o-dichlorobenzene, m-dichlorobenzene, p-dichlorobenzene, 1,2,4-trichlorobenzene, ethyl ether, tetrahydrofuran, pyridine, pyrrolidine, N-mehtylpyrrolidone N,N-dimethylformamide, and dimethyl sulfoxide, and more preferably, a species or a mixture of two or more species selected from methanol, benzene, toluene and xylene.

The amount of the solvent used is not particularly limited. However, in view of suppression of rapid heat generation during the reaction and the purity of the resultant ester amide monomer, the amount of the solvent for 1 mole of the diester compound and the diamine compound converted based on their total mole number is normally 0.1IL-100L and, preferably, 0.2L-50L and, more preferably, 0.3L-30L.

The heating temperature in a case where an organic solvent is used is not particularly limited. Normally, the heating may be performed at a temperature close to the boiling point of the solvent used.

The order of addition of chemicals used is not particularly limited. The chemicals may be simultaneously added or individual chemical ingredients may be sequentially added.

The reaction time remarkably varies depending on the reaction temperature and the amount of the solvent used. However, the reaction time is normally 0.1-50 hours and, preferably, 0.2-20 hours and, more preferably, 0.5-10 hours.

This reaction may be performed without using an organic solvent. In such a case, the heating temperature is preferably equal to or higher than the melting point of the diester compound but lower than or equal to the melting point of the ester amide monomer, in view of the purity of the resultant ester amide monomer.

In that case, there is no particular limitation on the order of addition of the chemicals used. The chemicals may be added simultaneously, or the individual chemical ingredients may be sequentially added.

As for the ester amide monomer thus produced, it is possible to wash the recovery obtained through filtration of the reaction solution or the reaction melt, with a solvent, for use. Furthermore, after washing with a solvent, vacuum drying may be performed for use. The solvent to be used for the washing varies depending the structure of the resultant ester amide monomer. However, it is possible to use, for example, methanol, ethanol, propanol, acetone, chloroform, benzene, toluene, water and the like. Furthermore, it is also possible to purify a product obtained by the filtration of the reaction solution and the subsequent washing with a solvent, by re-crystallization using a suitable solvent, before use. The re-crystallization solvent is not particularly limited as long as the solvent dissolves the ester amide monomer when heated and causes the ester amide monomer to precipitate when cooled. For example, a species or a mixture of two or more species selected from methanol, ethanol, propanol, dimethyl sulfoxide, N,N-dimethylformamide, N-methylpyrrolidone, chloroform and phenol may be used. The solvent is preferably a species or a mixture of two or more species selected from dimethyl sulfoxide, N,N-dimethylformamide, N-methylpyrrolidone, phenol and ethanol, and more preferably, a species or a mixture of two or more species selected from dimethyl sulfoxide, N,N-dimethylformamide, phenol and ethanol.

The polyester amide copolymer of the present invention may be compounded with other ingredients to produce a composition.

By further adding a filler to the polyester amide resin of the present invention, the mechanical properties and heat resistance can be further improved.

As the filler used according to the present invention, fibrous fillers such as aramid fiber, polyamide fiber, glass fiber, carbon fiber, alumina fiber, silicon carbide fiber, boron-based fiber, zirconia fiber, potassium titanium fiber, asbestos fiber, gypsum fiber, brass fiber, stainless steel fiber, steel fiber, ceramic fiber, and the like, and non-fibrous fillers such as graphite, wollastenite, glass flakes, glass beads, glass micro-balloons, molybdenum disulfide and the like, may be cited. The filler is preferably glass fiber, carbon fiber, alumina fiber, glass flakes and glass beads, and more preferably, glass fiber. Among the glass fibers, chopped strand-type glass fibers may be preferably used. The amount of the filler

added is 1-200 parts by weight relative to 100 parts by weight of the polyester amide resin, and preferably, 3-180 parts by weight, and more preferably, 5-150 parts by weight.

There is no particular limitation on the particle size and fiber length of the filler. If the filler is a particle-shape material, the particle size is preferably about 0.01-100 μm and, particularly preferably, 01-30 μm and, more preferably, 0.5-10 μm.

If the filler is a fibrous material, the fiber diameter is normally 1-30 μm, and preferably 2-25 μm, and more preferably 3-20 μm, and the fiber length is preferably 1 μm-20 mm, and particularly preferably 2 μm-15 mm, and more preferably 3 μm-10 mm.

The polyester amide copolymer of the present invention has a good compatibility specific to elastomers having glass transition temperatures equal to or lower than 20°C. By further adding such an elastomer, the impact characteristic is specifically improved, thereby providing a toughness.

The elastomer having a glass transition temperature of 20°C or lower is preferably olefin-based elastomer, nylon-based elastomer, polyester-based elastomer, polyester polyether-based elastomer, polyester-polyester-based elastomer, polyester polyamide-based elastomer other than that of the present invention, and the like, and more preferably, olefin-based elastomer. Cited as specific examples of the olefin-based elastomer are ethylene-propylene copolymer, ethylene-1-butene copolymer, ethylene-propylene-conjugated diene copolymer, ethylene-ethyl acrylate copolymer, ethylene-methacrylic acid copolymer, ethylene-glycidyl methacrylate copolymer, ethylene-vinyl acetate-glycidyl methacrylate copolymer, ethylene-ethyl acrylate-g-maleic anhydride copolymer, ethylene-methyl acrylate-g-maleic anhydride copolymer, ethylene-ethyl acrylate-g-maleimide copolymer, ethylene-ethyl acrylate-g-N-phenyl maleimide copolymer, ethylene-propylene, ethylene-butene-1-g-maleic anhydride copolymer, ethylene-propylene-1,4-hexadiene-g-maleic anhydride copolymer, ethylene-propylene-dichloropentadiene-g-maleic anhydride copolymer, ethylene-propylene-2,5-norbornadiene-g-maleic anhydride copolymer, ethylene-propylene-g-N-phenyl maleimide copolymer, styrene-maleic anhydride copolymer, styrene-butadiene-styrene-g-maleic anhydride block copolymer, styrene/ethylene-butylene/styrene-g-maleic anhydride block copolymer obtained by adding hydrogen to styrene-butadiene-styrene block copolymer and then grafting maleic anhydride, styrene- isoprene-g-maleic anhydride block copolymer, ethylene-acrylic acid ionomer, ethylene-methacrylic acid ionomer, ethylene-itaconic acid ionomer, and the like. These may be used individually or in the form of a mixture.

The amount of the elastomer having a glass transition temperature of 20°C of lower is preferably 1-100 parts by weight, and particularly preferably, 3-90 parts by weight, and more preferably 5-80 parts by weight, relative to 100 parts by weight of the polyester amide copolymer.

If the elastomer is used in a relatively small amount, for example, 50 parts by weight or less, and preferably, 30 parts by weight of less, and more preferably 20 parts by weight or less, the elastomer in the resin composition is present as a dispersed phase in the polyester amide resin. Fine dispersion is preferable in order for a form or molding produced from the composition of the present invention to have an excellent impact strength. A method for evaluating the mixed condition in the resin composition is a method wherein the particle size in the dispersed phase is used as an evaluation scale. If the polyester amide resin composition of the present invention is compounded with an elastomer, the average dispersion of the elastomer portion is preferably at most 15 microns, and more preferably, at most 10 microns.

In the polyester amide copolymer or the polyester amide resin composition of the present invention, the crystallization speed can be freely controlled by adding a crystal nucleus agent, and the thermal stability, the non-tinting characteristic, mechanical properties and the forming-processability are improved.

The crystal nucleus agent is not particularly limited as long as it is a compound that accelerates the crystallization of the polyester amide copolymer. Talc, mica, kaolin, silica, clay, metal oxides, carbonic acid salts, sulfuric acid salts, organic carboxylic acid salts, and organic sulfonic acid salts, and the like may be preferably used.

As a metal oxide, metal oxides having metals of groups II-V in the periodic table as center atoms may be used. Specifically, magnesium oxide, potassium oxide, barium oxide, titanium oxide, nickel oxide, copper oxide, zinc oxide, antimony oxide, aluminum oxide and the like may be used.

As a carbonic acid salt, carbonic acid salts of metals of groups I and II in the period table may be used. Specifically, sodium carbonate, potassium carbonate, calcium carbonate, barium carbonate, zinc carbonate, copper carbonate and the like may be used.

As a sulfuric acid salt, sulfuric acid salts of metals of groups I and II in the periodic table may be used. Specifically, sodium sulfate, potassium sulfate, calcium sulfate, barium sulfate, zinc sulfate, copper sulfate and the like may be used.

As an organic carboxylic acid salt, carboxylic acid salts having metals of groups I and II in the period table may be used. Specifically, lithium caproate, sodium caproate, potassium caproate, calcium caproate, magnesium caproate, barium caproate, zinc caproate, lithium stearate, sodium stearate, potassium stearate, calcium stearate, magnesium stearate, barium stearate, zinc stearate, lithium montanate, sodium montanate, potassium montanate, calcium montanate, magnesium montanate barium montanate, zinc montanate, lithium benzoate, sodium benzoate, potassium benzoate, calcium benzoate, magnesium benzoate barium benzoate, zinc benzoate, lithium terephthalate, sodium terephthalate, potassium terephthalate, calcium terephthalate, magnesium terephthalate barium terephthalate, zinc

terephthalate, and the like, may be used.

As an organic sulfonic acid salt, organic sulfonic acid salts of metals of groups I and II in the periodic table may be used. For example, lithium benzenesulfonate, sodium benzenesulfonate, potassium benzenesulfonate, p-lithium sulfobenzoate, p-sodium sulfobenzoate, p-potassium sulfobenzoate, sodium 2,5- or 3,5-dicarboxybenzenesulfonate, lithium 2,5- or 3,5-dicarboxybenzenesulfonate, potassium 2,5- or 3,5-dicarboxybenzenesulfonate, and the like, may be used.

The amount of the crystal nucleus agent used is normally 0.01-20 parts by weight, and preferably, 0.02-15 parts by weight, and more preferably, 0.03-10 parts by weight, relative to 100 parts by weight of the polyester amide copolymer.

The polyester amide copolymer or the polyester amide resin composition of the present invention can be improved in the heat resistance and heat stability by optionally further adding a heat resistant stabilizing agent, thereby remarkably improving the processability into resins, films and fibers. Moreover, besides the non-tinting characteristic of the processed article, odor during the processing can be suppressed.

As a heat resistant stabilizing agent according to the present invention, a species or a mixture of two or more species selected from hindered phenol-based antioxidants and metal hypophosphite salts may preferably be used. In particular, if a metal hypophosphite salt is added, the color tone of the composition can be remarkably improved.

Specific examples of the hindered phenol-based antioxidants that may be used are triethylene glycol-bis[3-(3-t-butyl-5-methyl-4-hydroxyphenyl) propionate],1,6-hexanediol-bis[3-(3,5-di-t-butyl-4-hydroxyphenyl) propionate], pentaerythrityl-tetrakis[3-(3,5-di-t-butyl-4-hydroxyphenyl) propionate], 2,2-thi-diethylene bis[3-(3,5-di-t-butyl-4-hydroxyphenyl) propionate], octadecyl-3-(3,5-di-t-butyl-4-hydroxyphenyl) propionate, 3,5-di-t-butyl-4-hydroxybenzyl phosphonate diethyl ester, 1,3,5-trimethyl-2,4,6-tris(3,5-di-t-butyl-4-hydroxybenzyl)benzene, bis or tris(3-t-butyl-6-methyl-4-hydroxyphenyl)propane, N,N'-hexamethylene bis(3,5-di-t-butyl-4-hydroxy-hydrocinnamide), N,N'-trimethylene bis(3,5-di-t-butyl-4-hydroxy-hydrocinnamide), 2-t-butyl-6-(3'-t-butyl-5'-methyl-2'-hydroxybenzyl)-4-methylphenyl acrylate, 3,9-bis[2-{3-(3-t-butyl-4-hydroxy-5-methylphenyl)propionyloxy}-1,1-dimethylethyl]-2,4,8,10-tetraoxaspiro[5,5]undecane, and the like.

As specific examples of metal hypophosphite salts, sodium hypophosphite, potassium hypophosphite, calcium hypophosphite, manganese hypophosphite, and the like, may be used.

The amount of the heat resistant stabilizing agent used according to the present invention is normally 0.01-30 parts by weight, and preferably, 0.02-25 parts by weight, and more preferably, 0.03-20 parts by weight, relative to 100 parts by weight of the polyester amide copolymer.

The polyester amide copolymer or the polyester amide resin composition of the present invention can be improved in the heat resistance and heat stability, by further adding a weathering-resistant stabilizing agent, thereby improving the non-tinting characteristic of the processed product during a long period of outdoor use.

As a weathering-resistant stabilizing agent according to the present invention, hindered amine-based compounds may preferably be used.

Specific examples of the hindered amine-based compounds that may be used are bis(1,2,2,6,6-pentamethyl-4-piperidiny l) sebacate, dimethyl succinate/1-(2-hydroxylethyl)-4-hydroxy-2,2,6,6,-tetramethylpiperidine polycondensation, N,N'-bis(3-amidopropyl)ethylenediamine/2,4-bis[N-butyl-N-(1 ,2,2,6,6-pentamethyl-4-piperidinyl)amino]-6-chloro-1,3,5-tr iazine polycondensation, poly[6-(1,1,3,5-tetramethylbutyl)a mino-1,3,5-triazine-2,4-diyl}{2,2,6,6,-tetramethyl-4-piperi dinyl)imino}hexamethylene{(2,2,6,6-tetramethyl-4-piperidyl) imino}], bis(2,2,6,6-tetramethyl-4-piperidyl) sebacate, and the like.

The amount of the weathering-resistant stabilizing agent added is normally 0.01-30 parts by weight, and preferably, 0.02-25 parts by weight, and more preferably, 0.03-20 parts by weight, relative to 100 parts by weight of the polyester amide copolymer.

The polyester amide copolymer or the polyester amide resin composition of the present invention can be improved not only in heat stability and impact characteristics but also in the suppression of order during the processing and the fluidity during film processing, by optionally adding an epoxy compound.

The epoxy compound according to the present invention is not particularly limited as long as the epoxy compound contains an epoxy group in its molecule. Monoepoxy compounds, diepoxy compounds and triepoxy compounds may preferably be used, and diepoxy compounds may be particularly preferably used. Specific examples of the epoxy compounds that may be used are diepoxy compounds represented by the following general formulas (28), (29), (30), (31), (32), (33), (34) and (35):

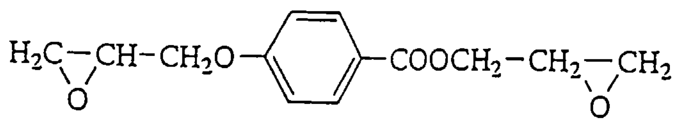 (28)

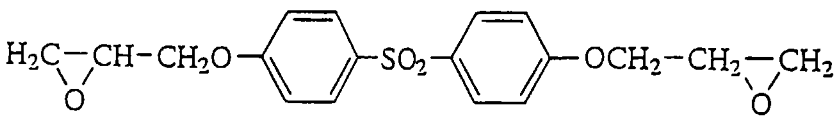 (29)

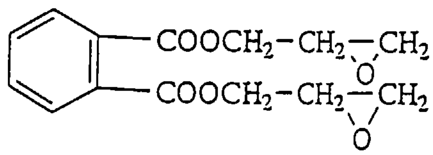 (30)

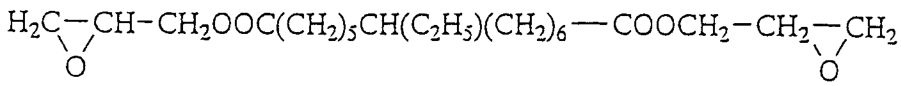 (31)

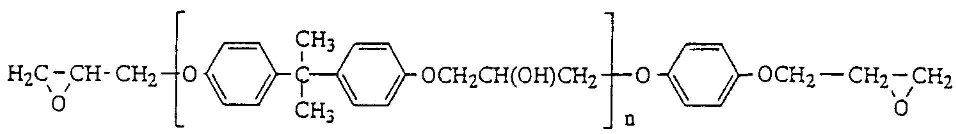 (32)

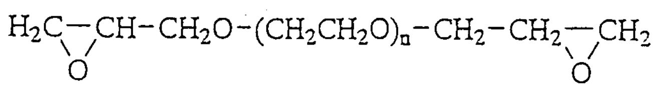 (33)

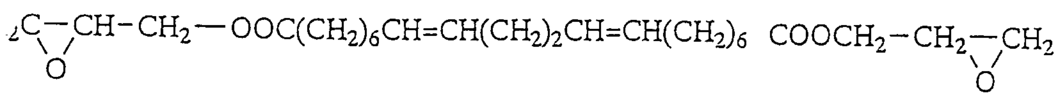 (34)

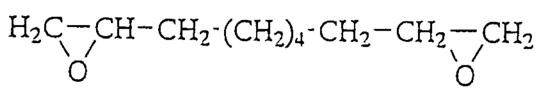 (35)

(n represents an integer equal to or greater than 1).

The amount of the epoxy compound added is normally 0.01-30 parts by weight, and preferably 0.02-25 parts by weight, and more preferably 0.03-20 parts by weight, relative to 100 parts by weight of the polyester amide copolymer.

Unlike the known resins, the polyester amide copolymer or the polyester amide resin composition of the present invention are remarkably excellent in compatibility with various resins, so that it is possible to add one or more species of thermoplastic resins other than the elastomers having glass transition temperatures of 20°C or lower to the polyester amide copolymer or the polyester amide resin composition of the present invention. Such thermoplastic resins are not particularly limited as long as they are synthetic resins which are other than the elastomers having glass transition temperatures of 20°C or lower, and exhibit fluidity when heated, and can be formed or molded utilizing this characteristic. For example, a species or a mixture of two or more species selected from polyester, all the aromatic polyesters, liquid crystal polyester, polycarbonate, polyamide, all the aromatic polyamides, phenoxy resin, polyketone, polyether ether ketone, polyether ketone, phenol resin, phenol-formaldehyde resin, polyallyl ether, polyamideimide, polyallyl sulfone,

polysulfone, ABS resins, acrylic resin, polyimide, polyphenylene sulfide and polyacetal, and olefin-based polymers such as polypropylene, polyethylene, polystyrene, ABS resin, acrylic resin, polyvinyl chloride and the like, and copolymers thereof, and the like, may be cited.

The amount of the thermoplastic resin added is normally 0.1-1000 percents by weight, and preferably 1-800 parts by weight, and more preferably 10-500 parts by weight, relative to 100 parts by weight of the polyester amide copolymer.

Furthermore, it is possible to add to the polyester amide copolymer or the polyester amide resin composition of the present invention, one or more species of additives that include other heat stabilizing agents, UV absorbing agents such as benzophenon-based UV absorbing agents and the like, antistatic agents such as amine-based antistatic agents and the like, lubricants such as amides, esters of aliphatic alcohols, and the like, bromine-based or phosphorus-based flame-retardant agents, mold releasing agents, lubricants, modifying agents such as ethers of polyalcohols and the like, coloring agents including dyes and pigments, coupling agents such as isocyanate-based coupling agents and the like, and the like.

If such a compound is compounded, the amount added is normally 0.01-30 parts by weight, and preferably, 0.02-25 parts by weight, and more preferably, 0.03-20 parts by weight, relative to 100 parts by weight of the polyester amide copolymer.

The polyester amide copolymer of the present invention has a great molecular weight and excellent heat resistance, heat stability and crystallinity, and is capable of being melt-formed, so that extrusion forming or molding, injection forming or molding, press forming or molding thereof can be performed. Therefore, it is possible to process the polyester amide copolymer into films, tubes, rods, fibers, and other forms having any desired shape and size. The polyester amide copolymer of the present invention is suitable particularly for processing of films and fibers wherein the dwell time during the processing is long.

EXAMPLES

The effects and advantages of the present invention will be described in detail hereinafter in conjunction with Examples. However, the present invention is not limited in any manner by the examples below.

The measurement items described with Examples and Comparative Examples were performed in accordance with the following methods. The term part or parts herein always indicates part or parts by weight. The method of measuring properties or characteristics are as follows.

(1) Number Average Molecular Weight (Mn) and Purity of Ester Amide Monomer

For measurement, gel permeation chromatography (GPC) was used. The measurement was performed using MODEL 510 high speed liquid chromatography and a differential refractometer WATER S410 by WATERS Company as a detector, under measurement conditions that the eluant was hexafluoroisopropanol, the column temperature was 25°C, and 0.1 ml of a solution of a sample concentration of 1-2 mg/ml was injected. As for columns, one column of Ultrastyragel

Linear filled with polystyrene porous swell gel by WATERS Company, and two of 500Å were connected in series, and an eluant of 0.5 ml/min and a column pressure of 500 psi were set. The polymer molecular weight was determined through conversion based on comparison with a calibration curve by standard poly methyl methacrylate. The purity of the ester amide monomer was calculated from the area ratio of peaks that occurred.

(2) Melting Point, Heat of Melting (ΔH)

Using a PERKIN-ELMER DSC-7, an endothermic peak (Tm1) which was to be observed in measurement using a sample amount of 10 mg in a nitrogen atmosphere under a condition of temperature increase from -100 at a rate of 20°C/min, was observed. Subsequently, a temperature of Tm1 + 20°C was maintained for 10 minutes. An endothermic peak temperature (Tm2) was observed in another measurement under a condition of temperature increase at a rate or 20°C. From the endothermic peak area determined from the peak of the endothermic peak temperature (Tm2), a heat of melting (ΔH) was calculated. A melting point was determined from the peak top of the melting point peak.

(3) Structure and Purity of Ester Amide Monomer, and Structure of Polyester Amide Copolymer

The structure of ester amide monomers was measured using 1H-NMR spectra and deuterated hexafluoroisopropanol, at a temperature of 30°C, by accumulation of 10 thousand times. The purity of ester amide monomers was determined in accordance with the flowchart of Fig. 1, as described above. That is, 500.0 g of a sample was accurately measured out, and 1L of DMF was added thereto, and the mixture was stirred at 20°C for 5 hours. A DMF insoluble fraction was recovered through filtration or centrifugation, and re-dissolved in 1L of hexafluoroisopropanol (HFIP). The mixture was stirred at 20°C for 5 hours. Then, an HFIP insoluble fraction was removed by filtration or centrifugation. After the HFIP solution was evaporated, the weight (W) of the HFIP soluble fraction was measured. A purity was calculated as purity = W/500 × 100 (wt.%) . The purity determined by this method is hereinafter termed the purity by the solvent separation method.

Although the purity of ester amide monomers can also be calculated from an integrated intensity ratio in 1H-NMR, the measurement using the solvent separation method is preferred in order to measure the purity with an increased precision.

The structure of the polyester amide copolymers was measured using 13C-NMR spectra and deuterated hexafluoroisopropanol, at a temperature of 30°C, by accumulation of 50 thousand times.

In the case of ester amide copolymers represented by the foregoing general formula (6), the sum of the two signal intensities observed at 138 ppm and 133 ppm observed in a 13C-NMR spectrum is determined as $\alpha$ (corresponding to the sequence represented by the foregoing general formula (7)), the signal intensity observed at 137 ppm is determined as $\beta$ (corresponding to the sequence represented by the foregoing general formula (8)), and the signal intensity observed at 134 ppm is determined as $\gamma$ (corresponding to the sequence represented by the foregoing general formula (9)).

The thus-calculated $\alpha$, $\beta$, $\gamma$ correspond to the mole percents (X, Y, Z) of the diad sequences represented by the foregoing general formulas (7), (8), (9), respectively. Therefore, the mole percents of the repetition units represented by the foregoing general formulas (7), (8), (9) can be calculated as $X = \alpha/(\alpha + \beta + \gamma) \times 100$, $Y = \beta/(\alpha + \beta + \gamma) \times 100$, and $Z = \gamma/(\alpha + \beta + \gamma) \times 100$. Among these equations, $X = \alpha/(\alpha + \beta + \gamma) \times 100$ indicates the alternate sequence mole percents. Therefore, by X, the alternating characteristic of the polyester amide copolymer was evaluated.

(4) Thermal Decomposition Temperature (Td5%: 5% weight reduction temperature)

Measurement was performed using a TG/TDA by SEIKO DENSHI Company, under conditions of a sample amount of 10 mg, a measurement temperature range of 100-850°C, a temperature increasing rate of 20°C/min, and a nitrogen atmosphere. With reference to the weight reduction curve obtained, the temperature at a 5% reduction of the weight was defined as Td5%.

(5) Moldability

Dumbbell test pieces of 1/2 type were molded at a mold temperature 60°C and a cylinder temperature set to the melting point of the polyester amide copolymer + 30°C, using a manually driven small-size molder, for evaluation of moldability. The moldability was evaluated as ○, if a dumbbell was formed by molding. If a test piece was so brittle that a dumbbell was not obtained, the moldability was evaluated as ×.

(6) Film Formability

Film formation capability was evaluated by melt-pressing at a temperature of the melting point of the polyester amide copolymer + 30°C. Evaluation ○ was given if the polymer was formed into a film shape by the melt pressing, and evaluation × was given if the polymer was so brittle that a film shape was not formed.

(7) Spinning Characteristic

400-500 d monofilaments were formed using a mouth piece having a mouth piece diameter of 1 mm-1.2 mm, at a cylinder temperature set to the melting point of the polyester amide copolymer + 30°C. The monofilaments were stretched at ratios of 8-10 times. Evaluation ○ was given if the polymer was formed into a fiber shape, and evaluation X was given if the polymer was so brittle that the polymer was not able to be spun into a fiber shape.

(8) Production of Polyester Amide Resin Composition and Preparation of Test Piece

Polyester amide copolymers according to the present invention were mixed with fillers and other necessary additives, and the mixtures were melted and kneaded at the melting point of the polyester amide copolymer + 30°C, using a 10-mm-$\Phi$ single shaft extruder. The pellets thereby obtained were dried and then supplied to a Sumitomo Nestal (phonetically translated) Injection Molder, PROMAT 40/25 (by SUMITOMO JUUKIKAI KOUGYOU KABUSHIKI GAISHA), whereby mold notch impact test pieces of 1/8" in thickness × 2 • 1/2" in length and tensile test pieces defined in ASTMD-638 were prepared. Using the test pieces, tensile strength, breaking extension, deflection temperature under a load of 1.82 MPa, and Izod impact strength were measured.

(9) Water Absorption Rate

The weight of tensile test pieces prepared by the aforementioned method, that is, the weight immediately after their formation, is determined as W0. The test pieces were left in water at room temperature and subjected to water absorption treatment. After 50 hours, the weight of the test pieces was measured. The measured weight is determined as W1. Using these values, a water absorption rate was calculated by the following equation:

$$\text{Water absorption rate} = (W1 - W0)/W0 \times 100$$

[PRODUCTION OF ESTER AMIDE MONOMER]

EXAMPLES 1-3, COMPARATIVE EXAMPLE 1: Production of Ester Amide Monomer

EXAMPLE 1: Production of Ester Amide Monomer (A-1) (Solution Method)

5 L of toluene solution containing 2220 g (12 mol) of dimethyl terephthalate and 0.1-fold mole of hexamethylenediamine, that is, in an amount of 140 g (1.2 mol), was provided in a three-neck flask to which a reflux tube and a thermometer were attached. After 140 g (5.9 wt.%) of titanium tetrabutoxy titanate was added as a catalyst, reaction was carried out under reflux for 6 hours. As the reaction progressed, a reactant precipitated. After the reaction, the reaction solution was heat-filtrated and the sediment was recovered. The obtained sediment was purified by recrystallization using N-dimethylformamide (DMF). Yield: 98% relative to hexamethylenediamine. The melting point measured by DSC: 238°C.

1H-NMR Spectrum, 7.6 ppm (4H: doublet, aromatic proton), 8.0 ppm (4H: doublet, aromatic proton), 6.6 ppm (2H: broad, NH), 3.9 ppm (6H: singlet, methoxy proton), 3.2-3.6 ppm (4H: broad, -CH2 NH-), 1.2-1.8 ppm (8H: broad, methylene)
IR Spectrum 3200-3600 cm-1 (NH expansion and contraction), 2800-3000 cm-1 (C-H expansion and contraction), 1720 cm-1 (C-O expansion and contraction), 1640 cm-1 (CO expansion and contraction, NH deflection), 1440 cm-1 (C-N expansion and contraction).

The above 1H-NMR spectrum and IR spectrum indicate that an ester amide monomer (A-1) represented by the following general formula (36) was obtained. No peak deriving from a compound other than the structure represented by the general formula was detected.
The purity of the obtained compound by the solvent separation method was 99.8%.

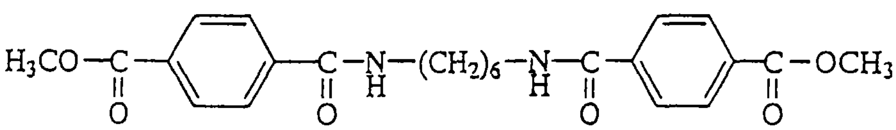

(36)

Fig. 2 and Fig. 3 show the 1H-NMR spectrum and the IR spectrum of the obtained ester amide monomer having a purity of 99.8%.

EXAMPLE 2

Production of Ester Amide Monomer (A-2) (Bulk Method)

2220 g (12 mol) of dimethyl terephthalate and 0.1-fold mole of hexamethylenediamine, that is, in an amount of 140 g (1.2 mol), were melted at a temperature of 150°C, which is equal to or higher than the melting point of dimethyl terephthalate but lower than or equal to the melting point of ester amide monomer in a three-neck flask to which a reflux tube and a thermometer were attached, and are reacted 140 g (5.9 wt.%) of titanium tetrabutoxy titanate for 6 hours. As the reaction progressed, a reactant precipitated. After the reaction, the reaction solution was heat-filtrated and the sediment was recovered. The obtained sediment was purified by recrystallization using DMF. Yield: 90% relative to hexamethylenediamine. The melting point measured by DSC: 238°C.

1H-NMR spectrum, 7.6 ppm (4H: doublet, aromatic proton), 8.0 ppm (4H: doublet, aromatic proton), 6.6 ppm (2H:

broad, NH), 3.9 ppm (6H: singlet, methoxy proton), 3.2-3.6 ppm (4H: broad, -CH2 NH-), 1.2-1.8 ppm (8H: broad, methylene)

IR spectrum 3200-3600 cm-1 (NH expansion and contraction), 2800-3000 cm-1 (C-H expansion and contraction), 1720 cm-1 (C-O expansion and contraction), 1640 cm-1 (CO expansion and contraction, NH deflection), 1440 cm-1 (C-N expansion and contraction).

The above 1H-NMR spectrum and IR spectrum indicate that an ester amide monomer (A-2) represented by the aforementioned general formula (36) was obtained. No peak deriving from a compound other than the structure represented by the general formula was detected.

The purity by the solvent separation method was 99.7%.

EXAMPLE 3

Production of Ester Amide Monomer (A-3)

Reaction was carried out in substantially the same manner as in Example 1, except that 140 g (5.9 wt.%) of ammonium chloride was used as a catalyst. As the reaction progressed, a reactant precipitated. After the reaction, the reaction solution was heat-filtrated and the sediment was recovered and reflux-washed with water for 5 hours. The obtained sediment was purified by recrystallization using DMF. Yield: 98% relative to hexamethylenediamine. The melting point measured by DSC: 238°C.

1H-NMR spectrum, 7.6 ppm (4H: doublet, aromatic proton), 8.0 ppm (4H: doublet, aromatic proton), 6.6 ppm (2H: broad, NH), 3.9 ppm (6H: singlet, methoxy proton), 3.2-3.6 ppm (4H: broad, -CH2 NH-), 1.2-1.8 ppm (8H: broad, methylene)

IR spectrum 3200-3600 cm-1 (NH expansion and contraction), 2800-3000 cm-1 (C-H expansion and contraction), 1720 cm-1 (C-O expansion and contraction), 1640 cm-1 (CO expansion and contraction, NH deflection), 1440 cm-1 (C-N expansion and contraction).

The above 1H-NMR spectrum and IR spectrum indicate that an ester amide monomer (A-1) represented by the aforementioned general formula (36) was obtained. No peak deriving from a compound other than the structure represented by the general formula was detected.

The purity of the obtained compound by the solvent separation method was 99.9%.

COMPARATIVE EXAMPLE 1

Production of Ester Amide Monomer (B-1)

Synthesis of an ester amide monomer was carried out by an ester amide monomer synthetic method (J. L. R. WILLIAMS, J. Org. Chem., 25,817 (1960)) adopted by J. L. R. WILLIAMS, J. Polym. Sci., 61,353 (1962).

1192 g (6 mol) of monomethyl terephthalic acid chloride was added to 5L of a pyridine solution containing 348 g (3 mol) hexamethylenediamine, while the solution was being stirred, in a three-neck flask to which a reflux tube and a thermometer were attached. After the addition was completed, the solution was stirred for 15 minutes. The reaction solution was then placed into an ice-water bath. A rough recovery was filtrated, and recrystallization from DMF was performed. The resultant needle crystal was recovered and vacuum-dried. Yield: 63.0%. The melting point measured by DSC: 232°C.

1H-NMR spectrum, 7.6 ppm (4H: doublet, aromatic proton), 8.0 ppm (4H: doublet, aromatic proton), 6.6 ppm (2H: broad, NH), 3.9 ppm (6H: singlet, methoxy proton), 3.2-3.6 ppm (4H: broad, -CH2 NH-), 1.2-1.8 ppm (8H: broad, methylene)

Impurity peak: 7.1 ppm (singlet, NH2)

IR spectrum 3200-3600 cm-1 (NH expansion and contraction), 2800-3000 cm-1 (C-H expansion and contraction), 1720 cm-1 (C-O expansion and contraction), 1640 cm-1 (CO expansion and contraction, NH deflection), 1440 cm-1 (C-N expansion and contraction).

Impurity peak: 1700 cm-1 (C-O expansion and contraction)

The above 1H-NMR spectrum and IR spectrum indicate that besides an ester amide monomer (A-1) represented by the aforementioned general formula (36), impurities represented by the following general formulas (37) and (38) were contained. The purity by the solvent separation method was 84.3%. Since the melting point was lower than that

of the ester amide monomers obtained by the methods of Examples 1, 2 and 3, it is also indicated that impurities were contained.

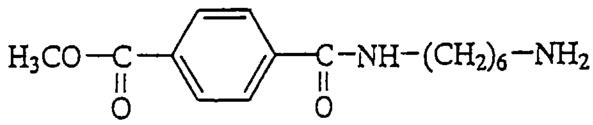

(37)

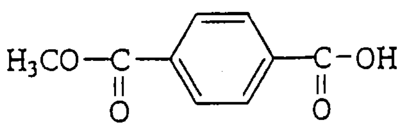

(38)

Although the ester amide monomer synthetic method (J. L. R. WILLIAMS, J. Org. Chem., 25,817 (1960)) adopted by J. L. R. WILLIAMS, J. Polym. Sci., 61,353 (1962) used an alcohol, such as a methanol, ethanol or the like, as a recrystallization solvent, the synthesized ester amide monomer (B-1) did not dissolve in any of these solvents, so that recrystallization could not be performed. Therefore, DMF was used as a recrystallization solvent. The purity of the monomer before the recrystallization by DMF was calculated based on the solvent separation method. The result was a very low purity of 70%. Thus, the reaction using the acid chloride produced large amounts of impurities and, despite the recrystallization, it was difficult to synthesize a high purity substance due to the low solubility of the impurities in the solvent.

These results clearly show that the methods according to the present invention produce high-purity ester amide monomers. On the other hand, the ester amide monomer obtained by the synthetic method adopted by J. L. R. WILLIAMS, J. Polym. Sci., 61,353 (1962) had a low purity even after it was subjected to recrystallization purification.

[PRODUCTION OF POLYESTER AMIDE COPOLYMER]

EXAMPLES 4, 5, COMPARATIVE EXAMPLE 2

Production of polyester amide copolymers were performed using the ester amide monomers (A-1, A-2, B-1) produced by the methods of Examples 1, 2 and Comparative Example 1 and a hexamethylene glycol as a glycol component.

Polymerization was performed using the ester amide monomer (A-1) synthesized in Example 1. The ester amide monomer (300 g, 681 mmol) and hexamethylene glycol (320 g, 2.73 mol) as a glycol component were weighed out into a condensation-polymerization test tube. After 6 mmol of tetra(n-butyl) titanate was added as a catalyst, the mixture was stirred at 260°C for 30 minutes under normal pressure and a nitrogen atmosphere. Then, a pressure reduction degree of 0.3 mmHg was achieved taking an hour. After that, a temperature increase to 280°C was accomplished taking another hour. After 3 hours, polymer was taken out of the test tube, washed in water, and dried under a reduced pressure, thus obtaining a polyester amide copolymer represented by the following general formula (39):

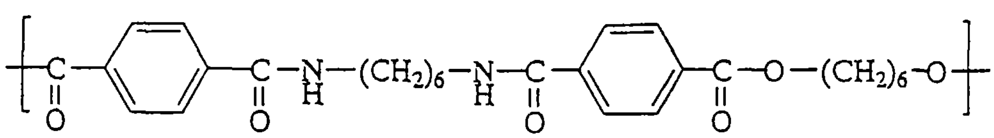

(39)

In Example 5, polymerization was performed using the ester amide monomer (A-2) synthesized in Example 2 and employing a similar method. In Comparative Example 2, polymerization was performed using the ester amide monomer (B-1) synthesized in Comparative Example 1 and employing a similar method.

A series of results are shown in Table 1.

[Table 1]

| Table 1 | Ester amide monomer | Glycol | Number average molecular weight Mn | Melting point of polymer (°C) | Melting heat (J/g) | Td (5%) | X (mol%) | Moldability | Film formability | Spinning charac-teristic |
|---|---|---|---|---|---|---|---|---|---|---|
| Example 4 | A-1 (Synth-esized article in Example 1) | Hexamethylene glycol | 15000 | 245 | 56 | 394 | 100 | ○ | ○ | ○ |
| Example 5 | A-2 (Synth-esized article in Example 2) | Hexamethylene glycol | 14500 | 245 | 54 | 395 | 100 | ○ | ○ | ○ |
| Comparative example 2 | B-1 (Synth-esized article in Comparative Example 1) | Hexamethylene glycol | 7000 | 265 No clear melting point exhibited | 25 | 350 | 60 | × | × | × |

Fig. 4 shows the 13C-NMR spectrum of the polyester amide copolymer obtained in Example 4.

In the signal regions of the aromatic carbons linked to carbonyls, only signals of 138 ppm and 133 ppm were observed. X = 100 mol% was obtained. It is indicated that the polyester amide copolymer was formed solely of alternate

diad sequences represented by the aforementioned general formula (7).

The 13C-NMR spectrum of the polyester amide copolymer obtained in Example 5 was substantially the same as the spectrum of the polyester amide copolymer obtained in Example 3, and X = 100% was obtained.

Fig. 5 shows the 13C-NMR spectrum of the polyester amide copolymer obtained in Comparative Example 2.

In the signal regions of the aromatic carbons linked to carbonyls, four signals of 138 ppm, 133 ppm, 137 ppm and 134 ppm were observed. That is, the copolymer contained not only the diad sequences represented by the aforementioned general formula (7) but also diad sequences represented by general formulas (8) and (9), and X = 60 mol% was obtained.

Through comparison of Examples 4, 5 and Comparative Example 2, it is shown that the polyester amide copolymer synthesized from high-purity ester amide monomers synthesized by methods according to the present invention are high molecular weight materials, and have excellent heat stability, high alternation characteristic, and high heat of melting thereby indicating a high crystallinity. On the other hand, as shown in Comparative Example 2, if the low-purity ester amide monomer synthesized in Comparative Example 1 is used, the resulting polymer has a low molecular weight, a poor heat resistance, and a poor alternation characteristic, and exhibits no clear melting point (that is, the melting point peak becomes broad), and a low heat of melting, thereby indicating a low crystallinity.

EXAMPLE 6, 7, COMPARATIVE EXAMPLE 3

In Example 6, polymerization was performed using the ester amide monomer (A-1) synthesize in Example 1. The ester amide monomer (300 g, 681 mmol) and ethylene glycol (100 g, 1.6 mol) as a glycol component were weighed out into a condensation-polymerization test tube. After 4 mmol of antimony trioxide was added as a catalyst, the mixture was stirred at 260°C for an hour under normal pressure and a nitrogen atmosphere. Then, a pressure reduction degree of 0.3 mmHg was achieved taking an hour. After that, a temperature increase to 300°C was accomplished taking another hour. After 2 hours, polymer was taken out of the test tube, washed in water, and dried under a reduced pressure, thus obtaining a polyester amide copolymer represented by the general formula (40). X = 100 mol% was obtained.

In Example 7, 2 mmol of manganese acetate was added, and the mixture was stirred at 260°C for an hour under normal pressure and a nitrogen atmosphere. After that, 4 mmol of antimony trioxide was added, and the mixture was stirred for another hour. Then, a pressure reduction degree of 0.3 mmHg was achieved taking an hour. After that, a temperature increase to 280°C was accomplished taking another hour. After 2 hours, polymer was taken out of the test tube, washed in water, and dried under a reduced pressure, thus obtaining a polyester amide copolymer represented by the general formula (40). X = 100 mol% was obtained.

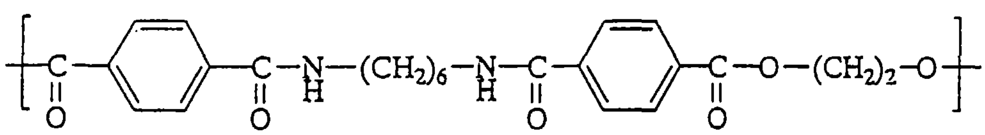
(40)

In Comparative Example 3, polymerization was performed using the ester amide monomer (B-1) synthesized in Comparative Example 1 and employing a method similar to that in Example 6. During the polymerization, the polymer melt decomposed into a dark brown color. The results are shown in Table 2.

[Table 2]

| Table 2 | Ester amide monomer | Glycol | Number average molecular weight Mn | Melting point of polymer (C) | Melting heat (J/g) | Td (5%) | X (mol%) | Moldability | Film formability | Spinning character-istic |
|---|---|---|---|---|---|---|---|---|---|---|
| Example 6 | A-1 (Synth-esized article in Example 1) | Ethylene glycol | 11000 | 265 | 38 | 393 | 100 | ○ | ○ | ○ |
| Example 7 | A-1 (Synth-esized article in Example 1) | Ethylene glycol | 50000 | 265 | 38 | 393 | 100 | ○ | ○ | ○ |
| Comparative example 3 | B-1 (Synth-esized article in Comparative Example 1) | Ethylene glycol | 4000 | 293 No clear melting point exhibited | 5 | 342 | 50 | × | × | × |

Through comparison of Example 6 and Comparative Example 3, it is shown that the polyester amide copolymer synthesized from an high-purity ester amide monomer synthesized by a method according to the present invention and ethylene glycol as a glycol component is a high molecular weight material, and has excellent heat stability, high alternation characteristic, and high heat of melting. On the other hand, as shown in Comparative Example 3, if the low-purity ester amide monomer synthesized in Comparative Example 1 is used, the resulting polymer has a low molecular weight, a poorer heat resistance, and a poor alternation characteristic, and exhibits no clear crystal peak.

Fig. 6 shows the 13C-NMR spectrum of the polyester amide copolymer obtained in Example 6.

In the signal regions of the aromatic carbons linked to carbonyls, only signals of 138 ppm and 133 ppm were observed. X = 100 mol% was obtained. It is indicated that the polyester amide copolymer was formed solely of alternate diad sequences represented by the aforementioned general formula (7).

The 13C-NMR spectrum of the polyester amide copolymer obtained in Example 7 was substantially the same as the spectrum of the polyester amide copolymer obtained in Example 6, and X = 100% was obtained.

Fig. 7 shows the 13C-NMR spectrum of the polyester amide copolymer obtained in Comparative Example 3.

In the signal regions of the aromatic carbons linked to carbonyls, four signals of 138 ppm, 133 ppm, 137 ppm and 134 ppm were observed. That is, the copolymer contained not only the diad sequences represented by the aforementioned general formula (7) but also diad sequences represented by general formulas (8) and (9), and X = 50 mol% was obtained.

EXAMPLE 8, COMPARATIVE EXAMPLE 4

In Example 8, polymerization was performed using the ester amide monomer (A-2) synthesized in Example 2. The ester amide monomer (300 g, 681 mmol) and 1,4-butanediol (180 g, 2.0 mol) as a glycol component were weighed out into a condensation-polymerization test tube. After 4 mmol of tetra(n-butyl) titanate was added as a catalyst, the mixture was stirred at 260°C for an hour under normal pressure and a nitrogen atmosphere. Then, a pressure reduction degree of 0.3 mmHg was achieved taking an hour. After that, a temperature increase to 290°C was accomplished taking another hour. After 2 hours, polymer was taken out of the test tube, washed in water, and dried under a reduced pressure, thus obtaining a polyester amide copolymer represented by the following general formula (21). X = 100 mol% was obtained. The results are shown in Table 3.

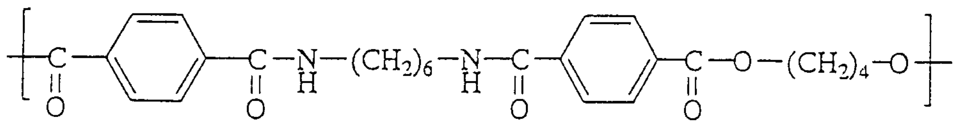

(41)

In Comparative Example 4, polymerization was performed using the ester amide monomer (B-1) synthesized in Comparative Example 1 and employing a method similar to that in Example 7. X = 60 mol% was obtained.

[Table3]

| Table 3 | Ester amide monomer | Glycol | Number average molecular weight Mn | Melting point of polymer (C) | Melting heat (J/g) | Td (5%) | X (mol%) | Moldability | Film formability | Spinning characteristic |
|---|---|---|---|---|---|---|---|---|---|---|
| Example 8 | A-1 (Synthesized article in Example 1) | Butanediol | 12000 | 250 | 49 | 392 | 100 | ○ | ○ | ○ |
| Comparative example 2 | B-1 (Synthesized article in Comparative Example 1) | Butanediol | 5000 | 300 No clear melting point exhibited | 12 | 348 | 60 | × | × | × |

Through comparison of Example 8 and Comparative Example 4, it is shown that the polyester amide copolymer synthesized from a high-purity ester amide monomer synthesized by a method according to the present invention and 1,4-butanediol as a glycol component is a high molecular weight material, and has excellent heat stability, high alternation characteristic, and a great amount of heat of melting, thereby indicating high crystallinity. On the other hand, as shown in Comparative Example 3, if the low-purity ester amide monomer synthesized in Comparative Example 1 is

used, the resulting polymer has a low molecular weight, a poor heat resistance, and a poor alternation characteristic, and has a small heat of melting, thereby indicating that it is a material of a poor crystallinity.

[PRODUCTION OF POLYESTER AMIDE RESIN COMPOSITION]

EXAMPLES 9-11

In Examples 9-11, the polyester amide copolymers produced in Example 4, Example 7 and Example 8 were mixed with glass fiber as a filler, and melted and kneaded using a 10-mm-Φ single shaft extruder. Since the polyester amide copolymers produced in Comparative Example 2, Comparative Example 3 and Comparative Example 4 had small molecular weights, and were poor in polymer heat stability, and decomposed during melting-kneading, it was difficult to produce resin compositions therefrom. Compounding formulation and results are shown in Table 1. The glass fibers used are chopped strands having a fiber length of 3 mm and a diameter of 13 μm.

[Table 4]

| Table 4 | Compounding formulation | | | Results | | | | | |
|---|---|---|---|---|---|---|---|---|---|
| | Polyester amide copolymer | Filler | | Tensile strength | Breaking extension | Izod impact value | Deflection temperature under load 1.82 MPa | Thermal decomposition starting temperature | Water absorption |
| | 100 parts by weight | Kind | Parts by weight | MPa | (%) | J/m | ( C) | ( C) | (%) |
| Example 9 | Polyester amide obtained in Example 4 | GF | 30 | 105 | 2.0 | 40 | 235 | 374 | 0.1 |
| Example 10 | Polyester amide obtained in Example 7 | GF | 30 | 120 | 2.0 | 35 | 245 | 370 | 0.1 |
| Example 11 | Polyester amide obtained in Example 8 | GF | 30 | 100 | 2.0 | 35 | 240 | 372 | 0.1 |

The results of Examples 9-11 indicate that articles formed of polyester amide resin compositions by mixing glass fiber as a filler, the structure of the polyester amide copolymer (regardless of the values of m, n), have high tensile strength, high impact strength and high deflection temperature under load. It is also indicated that they are low water-absorbing materials.

In Examples 12-17, the polyester amide copolymer synthesized in Example 4 was compounded with glass fiber and various additives.

EXAMPLES 12, 13

The polyester amide copolymer synthesized in Example 4 was compounded with glass fiber and ethylene-propylene rubber (modified EPR (MP0610) by MITSUI SEKIYU KAGAKU Company) or a glycidyl methacrylate-modified polyethylene copolymer (GMA-modified polyethylene copolymer (RA3050) by NIPPON SEKIYU KAGAKU Company) as an elastomer having a glass transition temperature of 20°C or lower. To measure the dispersion particle diameter of the elastomer in the obtained polyester amide resin composition, evaluation test pieces were sliced into thin sections by using an unltramicrotome, and photographs of the sections were taken by an optical microscope (transmitted light) and a transmission electron microscope. An average of 100 particles arbitrarily selected from the photographs was determined. The dispersion particle diameter of the elastomer in the polyester amide resin composition wax 1.6 μm or less. Particles were thus finely dispersed.

The compounding formulation and evaluation results are shown in Table 2. The results of Examples 12, 13 indicate that addition of an elastomer improves the breaking extension, the impact strength and the low water absorption without reducing the tensile strength or the deflection temperature under load.

EXAMPLE 14

The polyester amide copolymer synthesized in Example 4 was compounded with glass fiber and talc as a crystal nucleus agent. The compounding formulation and evaluation results are shown in Table 2. The results of Example 14 indicate that further compounding with talc further improves the tensile strength and the deflection temperature under load.

EXAMPLE 15

The polyester amide copolymer synthesized in Example 4 was compounded with glass fiber and a diepoxy compound represented by the following equation (28) as an epoxy compound. The compounding formulation and evaluation results are shown Table 2. The results of Example 15 indicate that further compounding with an epoxy compound improves the heat stability.

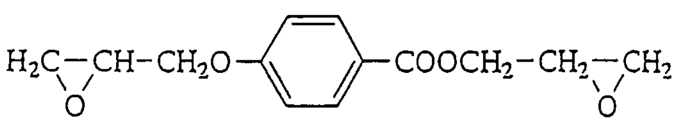

(28)

EXAMPLE 16

The polyester amide copolymer synthesized in Example 4 was compounded with glass fiber and hinder phenol-based antioxidant (triethylene glycol-bis[3-(3-t-butyl-5-methyl-4-hydroxyphenyl) propionate] ("Irganox" 245 by CIBA-GEIGY) as a heat resistant stabilizing agent and sodium hypophosphite as a metal hypophosphite salt. The compounding formulation and evaluation results are shown in Table 2. The results of Example 9 indicate that addition of a heat resistant stabilizing agent sodium hypophosphite improves the heat stability.

EXAMPLE 17

The polyester amide copolymer synthesized in Example 4 was compounded with glass fiber and hinder phenol-based antioxidant (triethylene glycol-bis[3-(3-t-butyl-5-methyl-4-hydroxyphenyl) propionate] ("Irganox" 245 by CIBA-GEIGY) as a heat resistant stabilizing agent, and sodium hypophosphite as a metal hypophosphite salt, and a hindered amine-based compound (bis(1,2,2,6,6-pentamethyl-4-piperidinyl) sebacate ("Sanol" LS765 SANKYO)), as a weathering-resistant stabilizing agent, and stearic acid amide as a mold releasing agent and a lubricant. The compounding formulation and evaluation results are shown in Table 2. The results of Example 17 indicate that addition of a heat resistant stabilizer, a weathering-resistant stabilizing agent, a mold releasing agent, and a lubricant improves the heat stability.

[Table 5]

| Table 5 | Compounding formulation | | | | | | | | | | | | Results | | | | | |
|---|---|---|---|---|---|---|---|---|---|---|---|---|---|---|---|---|---|---|
| | Polyester amide resin | Filler | | Elastomer | | Crystal nucleus agent | | Heat resistant stabilizer | Metal hypophosphite salt | Weathering resistant stabilizer | Mold releaser/Lubricant | Epoxy compound | Tensile strength | Breaking extension | Izod impact value | Deflection temperature under load 1.82 MPa | Thermal decomposition starting temperature | Water absorption |
| | 100 parts by weight | Kind | Parts by weight | Kind of elastomer | Parts by weight | Kind | Parts by weight | Parts by weight | Parts by weight | Parts by weight | Parts by weight | Parts by weight | MPa | (%) | (J/m) | ( C) | ( C) | (%) |
| Example 12 | Ester amide copolymer obtained in Example 4 | GF | 30 | MP0610 | 10 | | | | | | | | 114 | 3.0 | 50 | 235 | 374 | 0.03 |
| Example 13 | | GF | 30 | RA3050 | 10 | | | | | | | | 114 | 2.5 | 52 | 235 | 375 | 0.02 |
| Example 14 | | GF | 30 | | | talc | 0.01 | | | | | | 125 | 2.0 | 43 | 240 | 375 | 0.08 |
| Example 15 | | GF | 30 | | | | | | | | | 0.3 | 115 | 2.5 | 41 | 234 | 385 | 0.08 |
| Example 16 | | GF | 30 | | | | | 0.05 | 0.01 | | | | 115 | 2.0 | 41 | 234 | 387 | 0.08 |
| Example 17 | | GF | 30 | | | | | 0.05 | 0.01 | 0.1 | 0.1 | | 114 | 2.2 | 42 | 235 | 390 | 0.08 |

[Table 6]

| Table 6 | Compounding formulation | | | | | | | | | | | | Results | | | | | |
|---|---|---|---|---|---|---|---|---|---|---|---|---|---|---|---|---|---|---|
| | Polyester amide resin | Filler | | Elastomer | | Crystal nucleus agent | | Heat resistant stabilizer | Metal hypophosphite salt | Weathering resistant stabilizer | Mold releaser/Lubricant | Epoxy compound | Tensile strength | Breaking extension | Izod impact value | Deflection temperature under load 1.82 MPa | Thermal decomposition starting temperature | Water absorption |
| | 100 parts by weight | Kind | Parts by weight | Kind of elastomer | Parts by weight | Kind | Parts by weight | Parts by weight | Parts by weight | Parts by weight | Parts by weight | Parts by weight | MPa | (%) | (J/m) | ( C) | ( C) | (%) |
| Example 18 | Ester amide copolymer obtained in Example 7 | GF | 30 | MPO610 | 10 | | | | | | | | 130 | 3.0 | 45 | 245 | 370 | 0.03 |
| Example 19 | | GF | 30 | RA3050 | 10 | | | | | | | | 130 | 2.5 | 45 | 245 | 370 | 0.02 |
| Example 20 | | GF | 30 | | | talc | 0.01 | | | | | | 140 | 2.0 | 40 | 245 | 375 | 0.03 |
| Example 21 | | GF | 30 | | | | | | | | | 0.3 | 130 | 2.5 | 41 | 245 | 385 | 0.03 |
| Example 22 | | GF | 30 | | | | | 0.05 | 0.01 | | | | 132 | 2.0 | 41 | 245 | 387 | 0.03 |
| Example 23 | | GF | 30 | | | | | 0.05 | 0.01 | 0.1 | 0.1 | | 132 | 2.2 | 42 | 245 | 390 | 0.03 |

In Examples 18-23, resin compositions were produced in substantially the same manner as in Examples 12-17, except that the polyester amide copolymer synthesized in Example 7 was used. The results are shown in Table 6. The

above results indicate that compounding the polyester amide copolymer synthesized in Example 7 with glass fiber and various additives also improves the mechanical properties, the heat resistance and the heat stability as in the above examples.

INDUSTRIAL APPLICABILITY

A polyester amide copolymer and a composition containing the copolymer according to the present invention have high molecular weights and are excellent in heat resistance, heat stability, processability and crystallinity. Therefore, they can be used as engineering plastics, and can be molded or formed into various forms and, further, can be used as fibers, films.

Furthermore, according to the present invention, an ester amide monomer with a high purity can be obtained, so that a polyester amide copolymer having excellent properties as described above can be produced.

**Claims**

1. A polyester amide copolymer characterized in that the copolymer has a repeated structure substantially represented by general formula (1) and that if mole percentages of three diad sequences present in the polymer and represented by formulas (2), (3) and (4) are X, Y and Z (mol%) ( X + Y + Z = 100 ), the mole percentage of X in the polymer satisfies 70 ≤ X ≤ 100 (mol%):

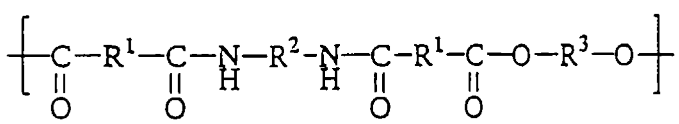

(1)

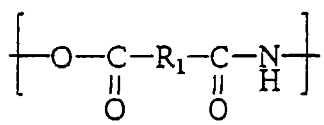

(2)

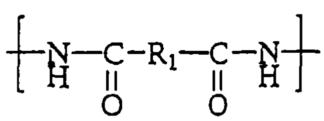

(3)

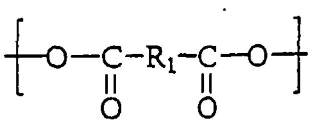

(4)

(where $R^1$, $R^2$ and $R^3$ represent bivalent substituted or non-substituted aliphatic group, alicyclic group or aromatic group).

2. A polyester amide copolymer characterized in that the copolymer has a repeated structure substantially represented by general formula (1) and that peak intensities α, β and γ based on diad sequence formulas (2), (3) and (4) which are observed in a $^{13}$C-NMR spectrum satisfy the following expression $70 \le \alpha/(\alpha + \beta + \gamma) \times 100 \le 100$ :

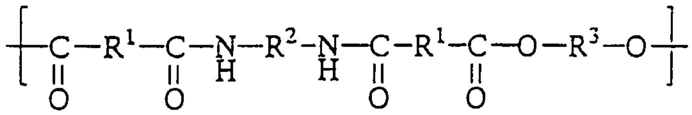
(1)

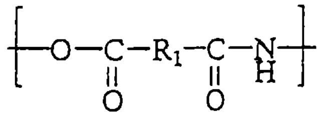
(2)

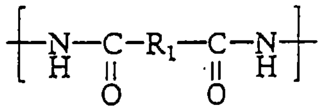
(3)

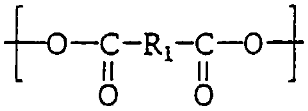
(4)

(where $R^1$, $R^2$ and $R^3$ represent bivalent substituted or non-substituted aliphatic group, alicyclic group or aromatic group).

**3.** A polyester amide copolymer according to claim 1, characterized in that in general formulas (1), (2), (3) and (4), $R^1$, $R^2$ and $R^3$ have one or more groups selected from the following general formulas (5):

$-(CH_2)_l-$ , 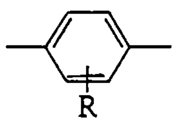 , 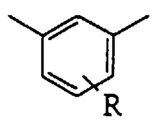 , 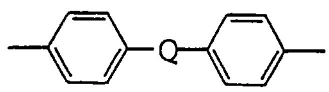 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

**4.** A polyester amide copolymer according to claim 1, characterized in that general formulas (1), (2), (3) and (4) are represented by general formulas (6), (7), (8) and (9):

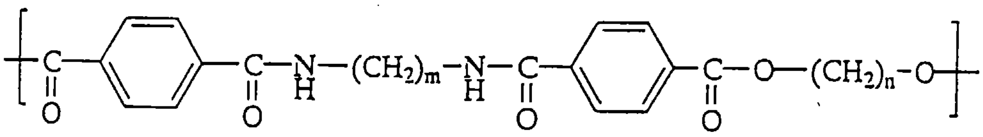 (6)

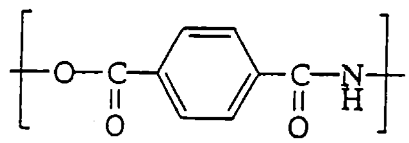 (7)

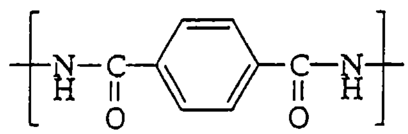 (8)

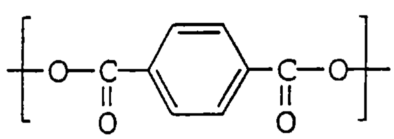 (9)

(where m and n represent integers equal to or greater than 2).

**5.** A polyester amide copolymer according to claim 2, characterized in that in general formulas (1), (2), (3) and (4), $R^1$, $R^2$ and $R^3$ have one or more groups selected from general formulas (5):

$-(CH_2)_l-$ , 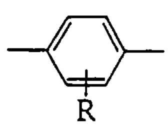 , 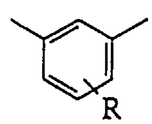 , 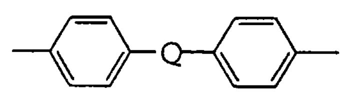 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

**6.** A polyester amide copolymer according to claim 2, characterized in that general formulas (1), (2), (3) and (4) are represented by general formulas (6), (7), (8) and (9):

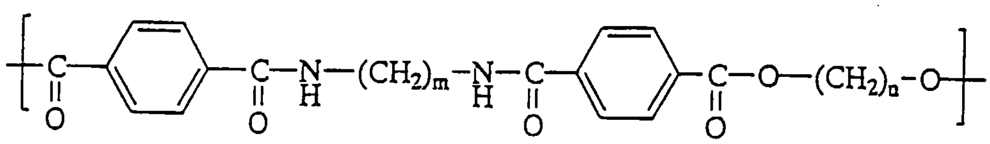
(6)

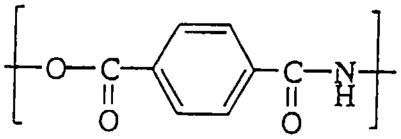
(7)

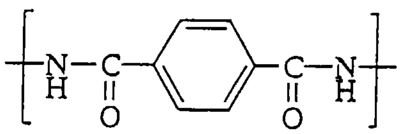
(8)

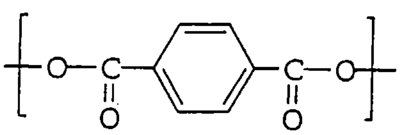
(9)

(where m and n in the above formulas represent integers equal to or greater than 2).

7. A polyester amide copolymer according to claim 1, characterized in that a number average molecular weight of the polyester amide copolymer measured by gel permeation chromatography and then converted based on a polymethylmethacrylate is equal to or greater than 10 thousand.

8. A polyester amide copolymer according to claim 3, characterized in that a number average molecular weight of the polyester amide copolymer measured by gel permeation chromatography and then converted based on a polymethylmethacrylate is equal to or greater than 10 thousand.

9. A polyester amide copolymer according to claim 4, characterized in that a number average molecular weight of the polyester amide copolymer measured by gel permeation chromatography and then converted based on a polymethylmethacrylate is equal to or greater than 10 thousand.

10. A polyester amide copolymer according to claim 2, characterized in that a number average molecular weight of the polyester amide copolymer measured by gel permeation chromatography and then converted based on a polymethylmethacrylate is equal to or greater than 10 thousand.

11. A polyester amide copolymer according to claim 5, characterized in that a number average molecular weight of the polyester amide copolymer measured by gel permeation chromatography and then converted based on a polymethylmethacrylate is equal to or greater than 10 thousand.

12. A polyester amide copolymer according to claim 6, characterized in that a number average molecular weight of the polyester amide copolymer measured by gel permeation chromatography and then converted based on a polymethylmethacrylate is equal to or greater than 10 thousand.

13. A polyester amide copolymer according to claim 1, characterized in that a 5 wt.% reduction temperature is 370°C

or higher.

**14.** A polyester amide copolymer according to claim 3, characterized in that a 5 wt.% reduction temperature is 370°C or higher.

**15.** A polyester amide copolymer according to claim 4, characterized in that a 5 wt.% reduction temperature is 370°C or higher.

**16.** A polyester amide copolymer according to claim 2, characterized in that a 5 wt.% reduction temperature is 370°C or higher.

**17.** A polyester amide copolymer according to claim 5, characterized in that a 5 wt.% reduction temperature is 370°C or higher.

**18.** A polyester amide copolymer according to claim 6, characterized in that a 5 wt.% reduction temperature is 370°C or higher.

**19.** A polyester amide copolymer according to claim 4, characterized in that $m = n = 6$ , and a quantity of heat of melting is 50 J/g or greater.

**20.** A polyester amide copolymer according to claim 6, characterized in that $m = n = 6$ , and a quantity of heat of melting is 50 J/g or greater.

**21.** A polyester amide copolymer according to claim 9, characterized in that $m = n = 6$ , and a quantity of heat of melting is 50 J/g or greater.

**22.** A polyester amide copolymer according to claim 12, characterized in that $m = n = 6$ , and a quantity of heat of melting is 50 J/g or greater.

**23.** A polyester amide copolymer according to claim 4, characterized in that $m = 6$, $n = 4$, and a quantity of heat of melting is 40 J/g or greater.

**24.** A polyester amide copolymer according to claim 6, characterized in that $m = 6$, $n = 4$, and a quantity of heat of melting is 40 J/g or greater.

**25.** A polyester amide copolymer according to claim 9, characterized in that $m = 6$, $n = 4$, and a quantity of heat of melting is 40 J/g or greater.

**26.** A polyester amide copolymer according to claim 12, characterized in that $m = 6$, $n = 4$, and a quantity of heat of melting is 40 J/g or greater.

**27.** A polyester amide copolymer according to claim 4, characterized in that $m = 6$, $n = 2$, and a quantity of heat of melting is 30 J/g or greater.

**28.** A polyester amide copolymer according to claim 6, characterized in that $m = 6$, $n = 2$, and a quantity of heat of melting is 30 J/g or greater.

**29.** A polyester amide copolymer according to claim 9, characterized in that $m = 6$, $n = 2$, and a quantity of heat of melting is 30 J/g or greater.

**30.** A polyester amide copolymer according to claim 12, characterized in that $m = 6$, $n = 2$, and a quantity of heat of melting is 30 J/g or greater.

**31.** A method of producing a polyester amide copolymer characterized by heating an ester amide monomer represented by general formula (10) and a glycol represented by general formula (11) in the presence of a catalyst at a polymerization temperature of 150-350°C, and then further heating them at a pressure reduction degree of 0.01-100 mmHg at a polymerization temperature of 150°C-350°C:

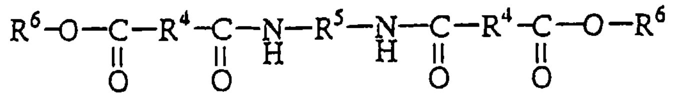 (10)

$$HO\text{-}R^7\text{-}OH \qquad (11)$$

(where $R^4$, $R^5$ and $R^7$ represent bivalent substituted or non-substituted aliphatic group, alicyclic group or aromatic group, and $R^6$ represents hydrogen or alkyl group of a carbon number of 1-5).

**32.** A method of producing a polyester amide copolymer according to claim 31, characterized in that $R^4$, $R^5$ and $R^7$ in the ester amide monomer represented by general formula (10) and the glycol represented by general formula (11) are one or more groups selected from general formulas (5):

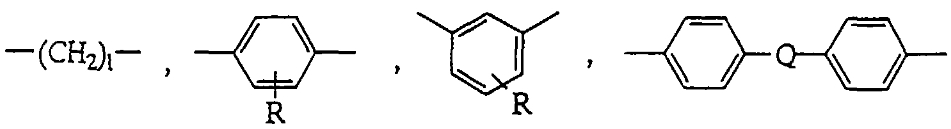 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group.

**33.** A method of producing a polyester amide copolymer according to claim 31, characterized in that the ester amide monomer represented by general formula (10) and the glycol represented by general formula (11) are represented by general formulas (12) and (13):

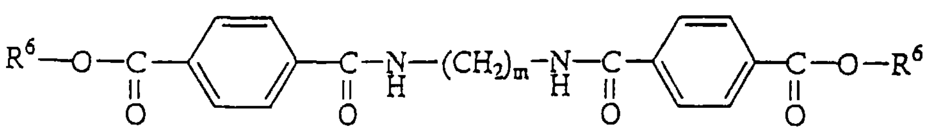 (12)

$$HO\text{-}(CH_2)_n\text{-}OH \qquad (13)$$

(where m and n represent integers equal to or greater than 2, and $R^6$ represents hydrogen or alkyl group of a carbon number of 1-5).

**34.** An ester amide monomer which is represented by general formula (10) and whose purity is 90 wt.% or higher:

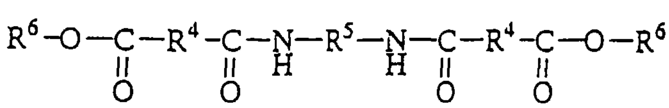 (10)

(where $R^4$ and $R^5$ represent bivalent substituted or non-substituted aliphatic group, alicyclic group or aromatic group, and $R^6$ represents hydrogen or alkyl group of a carbon number of 1-5).

**35.** An ester amide monomer according to claim 34, characterized in that $R^4$ and $R^5$ in the ester amide monomer represented by general formula (10) are one or more groups selected from general formulas (5):

$-(CH_2)_l-$ , 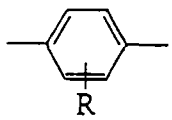 , 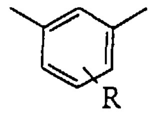 , 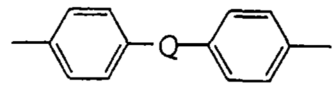 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

**36.** An ester amide monomer according to claim 34, characterized in that the ester amide monomer represented by general formula (10) is represented by general formula (12):

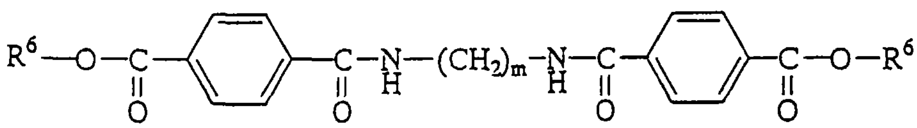 (12)

(where m represents an integer equal to or greater than 2, and $R^6$ represents hydrogen or alkyl group of a carbon number of 1-5).

**37.** An ester amide monomer which is represented by general formula (10) and whose purity is 95 wt.% or higher:

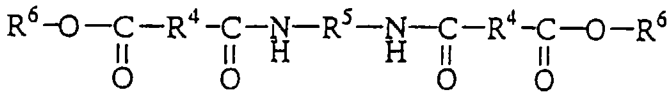 (10)

(where $R^4$ and $R^5$ represent bivalent substituted or non-substituted aliphatic group, alicyclic group or aromatic group, and $R^6$ represents hydrogen or alkyl group of a carbon number of 1-5).

**38.** An ester amide monomer according to claim 37, characterized in that $R^4$ and $R^5$ in the ester amide monomer represented by general formula (10) have one or more groups selected from general formulas (5):

$-(CH_2)_l-$ , 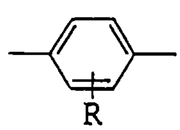 , 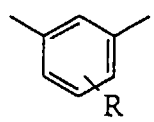 , 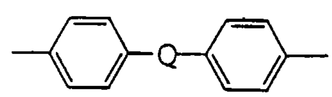 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

**39.** An ester amide monomer according to claim 37, characterized in that the ester amide monomer represented by general formula (10) is represented by general formula (12):

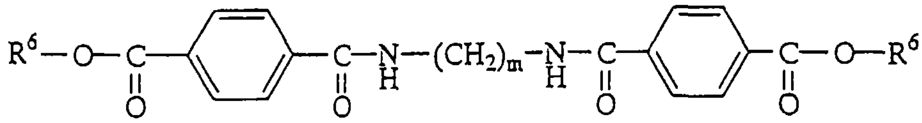 (12)

(where m represents an integer equal to or greater than 2, and $R^6$ represents hydrogen or alkyl group of a carbon number of 1-5).

**40.** A method of producing an ester amide monomer characterized by heating a diester compound represented by general formula (14) and a diamine compound represented by general formula (15) in the presence or absence of an organic solvent:

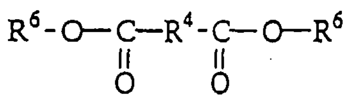 (14)

$$H_2N\text{-}R^5\text{-}NH_2 \qquad (15)$$

(where $R^4$ and $R^5$ represent bivalent substituted or non-substituted aliphatic group, alicyclic group or aromatic group, and $R^6$ represents hydrogen or alkyl group of a carbon number of 1-5).

**41.** A method of producing an ester amide monomer according to claim 40, characterized in that $R^4$ and $R^5$ in the diester compound represented by general formula (14) and the diamine compound represented by general formula (15) are one or more groups selected from general formulas (5):

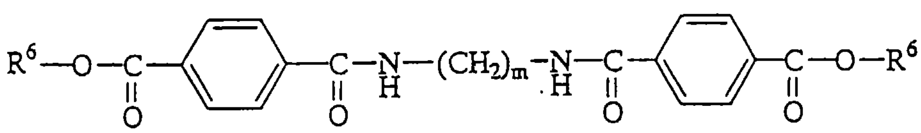 (5)

(where l represents an integer equal to or greater than 2, and R represents hydrogen, halogen or a monovalent organic residue, and Q represents a direct bond, O, S, $SO_2$, $C(CH_3)_2$, $CH_2$ or CHPh, Ph representing phenyl group).

**42.** A method of producing an ester amide monomer according to claim 40, characterized in that the diester compound represented by general formula (14) and the diamine compound represented by general formula (15) are a diester compound represented by general formula (16) and a diamine compound represented by general formula (17):

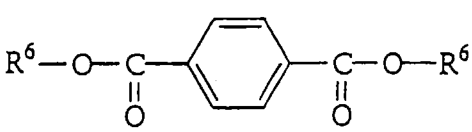 (16)

$$H_2N\text{-}(CH_2)_m\text{-}NH_2 \quad (17)$$

(where m represents an integer equal to or greater than 2, and $R^6$ represents alkyl group of a carbon number of 1-5).

**43.** A method of producing an ester amide monomer according to claim 40, characterized in that the organic solvent has a characteristic that the organic solvent dissolves the diester compound represented by general formula (14) and the diamine compound represented by general formula (15) but does not dissolve the ester amide monomer.

**44.** A method of producing an ester amide monomer according to claim 40, characterized in that the organic solvent has a characteristic that the organic solvent dissolves a diester compound represented by general formula (14) and a diamine compound represented by general formula (15) in which $R^4$ and $R^5$ are one or more groups selected from general formulas (5), but does not dissolve the ester amide monomer.

**45.** A method of producing an ester amide monomer according to claim 40, characterized in that the organic solvent has a characteristic that the organic solvent dissolves a diester compound represented by general formula (16) and a diamine compound represented by general formula (17) but does not dissolve the ester amide monomer.

**46.** A method of producing an ester amide monomer according to claim 40, characterized in that the diester compound represented by general formula (14) and the diamine compound represented by general formula (15) are heated in the absence of an organic solvent at a temperature equal to or higher than a melting point of the diester compound and lower than or equal to a melting point of the ester amide monomer.

**47.** A method of producing an ester amide monomer according to claim 40, characterized in that there is a characteristic in that among the diester compound represented by general formula (14) and the diamine compound represented by general formula (15), a diester compound and a diamine compound in which $R^4$ and $R^5$ are one or more groups selected from general formulas (5) are dissolved, but the ester amide monomer is not dissolved.

**48.** A method of producing an ester amide monomer according to claim 40, characterized in that a diester compound represented by general formula (16) and a diamine compound represented by general formula (17) are heated in the absence of an organic solvent at a temperature equal to or higher than a melting point of the diester compound and lower than or equal to a melting point of the ester amide monomer.

**49.** A method of producing an ester amide monomer according to claim 40, characterized in that the mole ratio of the diamine compound to the diester is equal to or greater than 0.01-fold mole but less than or equal to 0.3-fold mole.

**50.** A method of producing an ester amide monomer according to claim 40, characterized in that a species or a mixture of two or more species selected from monobutylhydroxy tinoxide, titanium tetrabutoxide, cesium fluoride, antimony fluoride, antimony oxides, metal alcoxides, ammonium salts, metal acetate salts, metal hydrides, organometallic compounds, alkaline inorganic compounds, and alkali metals, is used.

**51.** A method of producing a polyester amide copolymer according to claim 31, characterized in that the ester amide monomer is an ester amide monomer obtained by heating a diester compound represented by general formula (14) and a diamine compound represented by general formula (15) in the presence or absence of an organic solvent.

**52.** A method of producing a polyester amide copolymer according to claim 32, characterized in that the ester amide monomer is an ester amide monomer obtained by heating a diester compound represented by general formula (14) and a diamine compound represented by general formula (15) in which diester compound and diamine compound $R^4$ and $R^5$ are one or more groups selected from general formulas (5), in the presence or absence of an organic solvent.

**53.** A method of producing a polyester amide copolymer according to claim 33, characterized in that the ester amide monomer is an ester amide monomer obtained by heating a diester compound represented by general formula (16) and a diamine compound represented by general formula (17) in the presence or absence of an organic solvent.

**54.** A polyester amide resin composition which is obtained by further compounding a filler with 100 parts by weight of a polyester amide copolymer described in any one of claims 1-30, in an amount of 1-200 parts by weight relative to 100 parts by weight of the polyester amide copolymer.

**55.** A polyester amide resin composition which is obtained by further compounding an elastomer having a glass transition temperature of 20°C or lower with a polyester amide copolymer described in any one of claims 1-30, in an amount of 1-100 parts by weight relative to 100 parts by weight of the polyester amide copolymer.

**56.** A polyester amide resin composition according to claim 55, characterized in that the elastomer having a glass transition temperature of 20°C or lower is an olefin-based elastomer.

**57.** A polyester amide resin composition which is obtained by further compounding a crystal nucleus agent with a polyester amide copolymer described in any one of claims 1-30 or a polyester amide resin composition described in claim 54 or 55, in an amount of 0.01-20 parts by weight relative to 100 parts by weight of the polyester amide copolymer.

**58.** A polyester amide resin composition according to claim 54, wherein the crystal nucleus agent is a species or a mixture of two or more species selected from talc, mica, kalion, silica, clay, inorganic carboxylic acid salts, inorganic sulfonic acid salts, metal oxides, carbonic acid salts, organic carboxylic acid salts, and organic sulfonic acid salts.

**59.** A polyester amide resin composition which is obtained by further compounding a heat-resistant stabilizing agent with a polyester amide copolymer described in any one of claims 1-30 or a polyester amide resin composition described in claim 54, 55 or 57, in an amount of 0.01-30 parts by weight relative to 100 parts by weight of the polyester amide copolymer.

**60.** A polyester amide resin composition which is obtained by further compounding a weathering-resistant stabilizing agent with a polyester amide copolymer described in any one of claims 1-30 or a polyester amide resin composition described in claim 54, 55, 57 or 59, in an amount of 0.01-30 parts by weight relative to 100 parts by weight of the polyester amide copolymer.

**61.** A polyester amide resin composition which is obtained by further compounding a mold releasing agent with a polyester amide copolymer described in any one of claims 1-30 or a polyester amide resin composition described in claim 54, 55, 57, 59 or 60, in an amount of 0.01-30 parts by weight relative to 100 parts by weight of the polyester amide copolymer.

**62.** A polyester amide resin composition which is obtained by further compounding a lubricant with a polyester amide copolymer described in any one of claims 1-30 or a polyester amide resin composition described in claim 54, 55, 57, 59, 60 or 61, in an amount of 0.01-30 parts by weight relative to 100 parts by weight of the polyester amide copolymer.

**63.** A polyester amide resin composition which is obtained by further compounding an epoxy compound with a polyester amide copolymer described in any one of claims 1-30 or a polyester amide resin composition described in any one of claims 54, 55, 57 and 59-62, in an amount of 0.01-30 parts by weight relative to 100 parts by weight of the polyester amide copolymer.

**64.** A polyester amide resin composition which is obtained by further compounding a thermoplastic resin other than elastomers having glass transition temperatures of 20°C or lower, with a polyester amide copolymer described in any one of claims 1-30 or a polyester amide resin composition described in any one of claims 54, 55, 57 and 59-63, in an amount of 0.1-1000 parts by weight relative to 100 parts by weight of the polyester amide copolymer.

**65.** A form containing a polyester amide copolymer described in any one of claims 1-30 or a polyester amide resin composition described in any one of claims 54, 55, 57 and 59-64.

**66.** A fiber containing a polyester amide copolymer described in any one of claims 1-30 or a polyester amide resin composition described in any one of claims 54, 55, 57 and 59-64.

**67.** A film containing a polyester amide copolymer described in any one of claims 1-30 or a polyester amide resin composition described in any one of claims 54, 55, 57 and 59-64.

Fig. 1

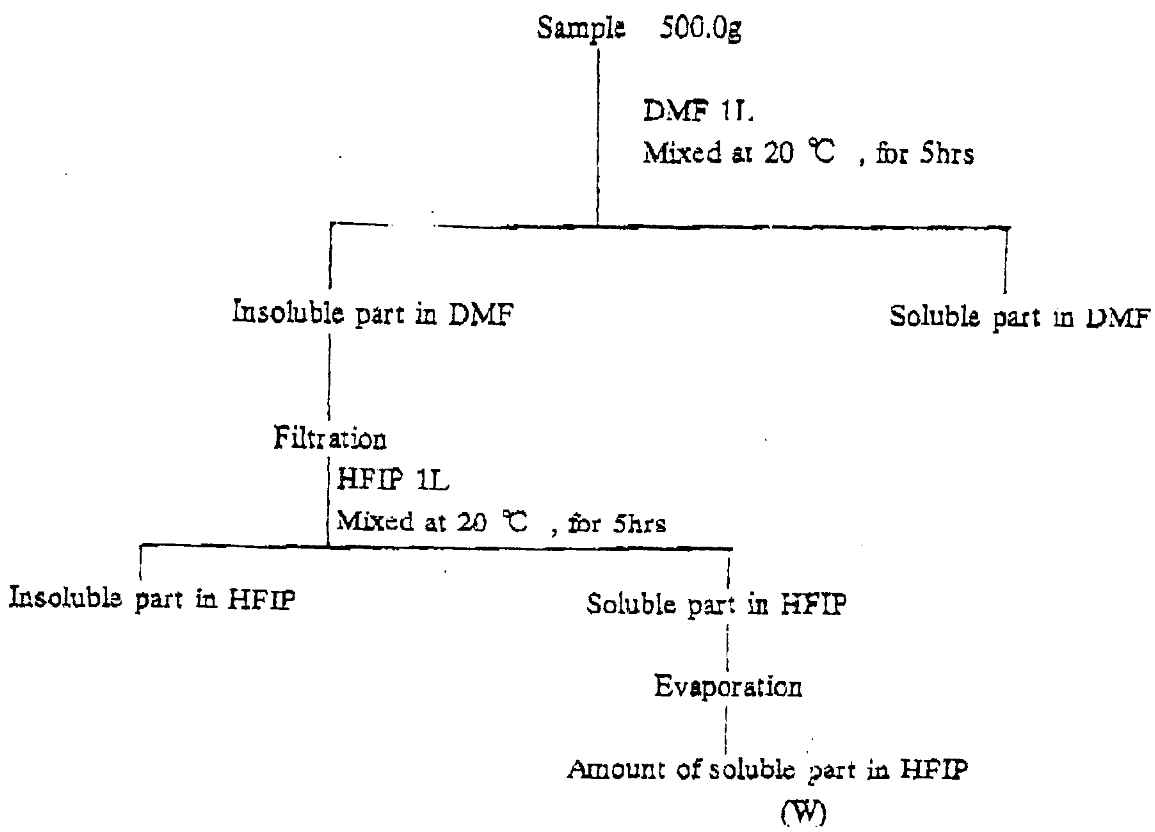
Sample 500.0g
DMF 1L
Mixed at 20 ℃ , for 5hrs
Insoluble part in DMF
Soluble part in DMF
Filtration
HFIP 1L
Mixed at 20 ℃ , for 5hrs
Insoluble part in HFIP
Soluble part in HFIP
Evaporation
Amount of soluble part in HFIP
(W)

Fig. 2

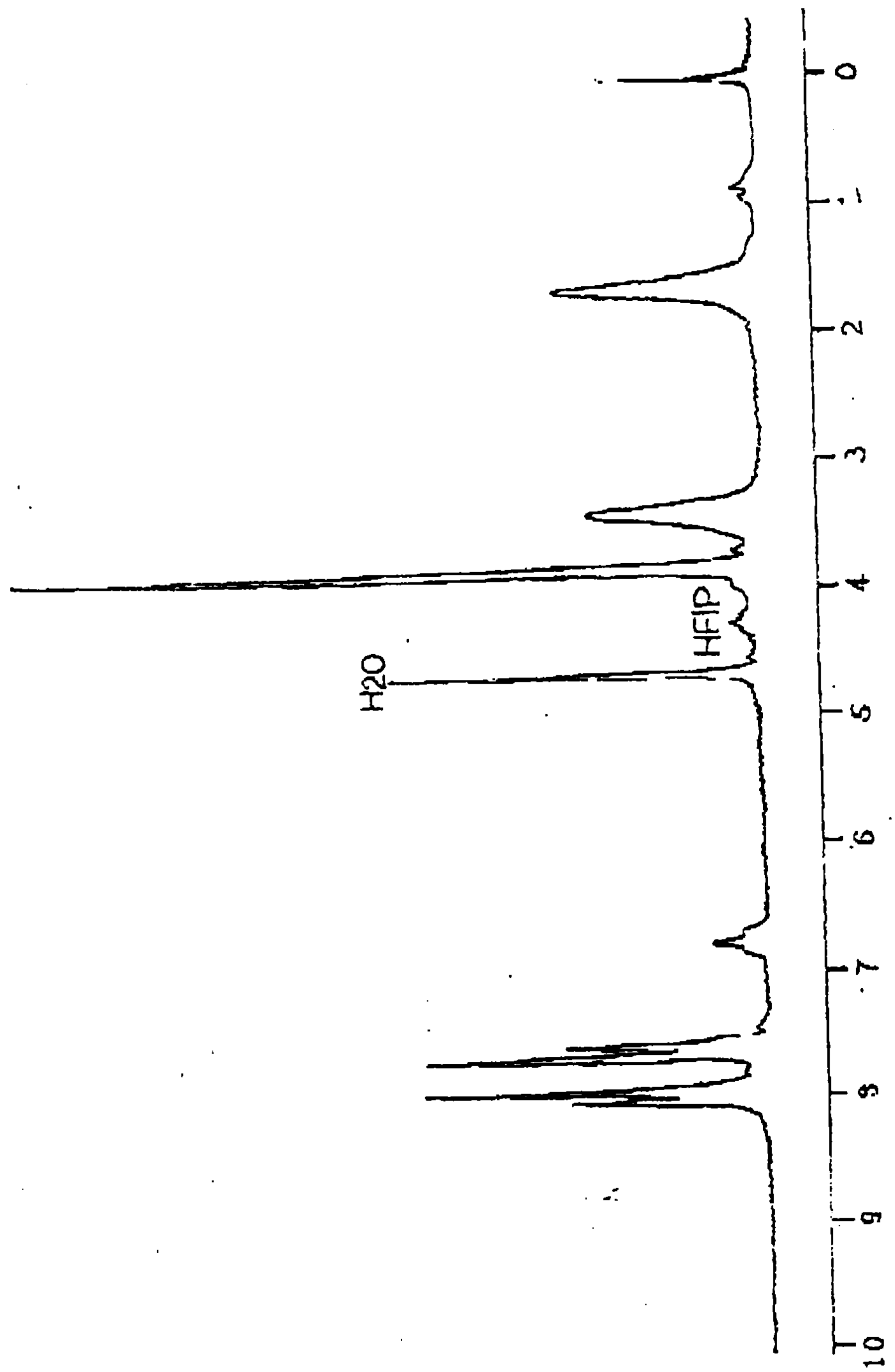

Fig. 3

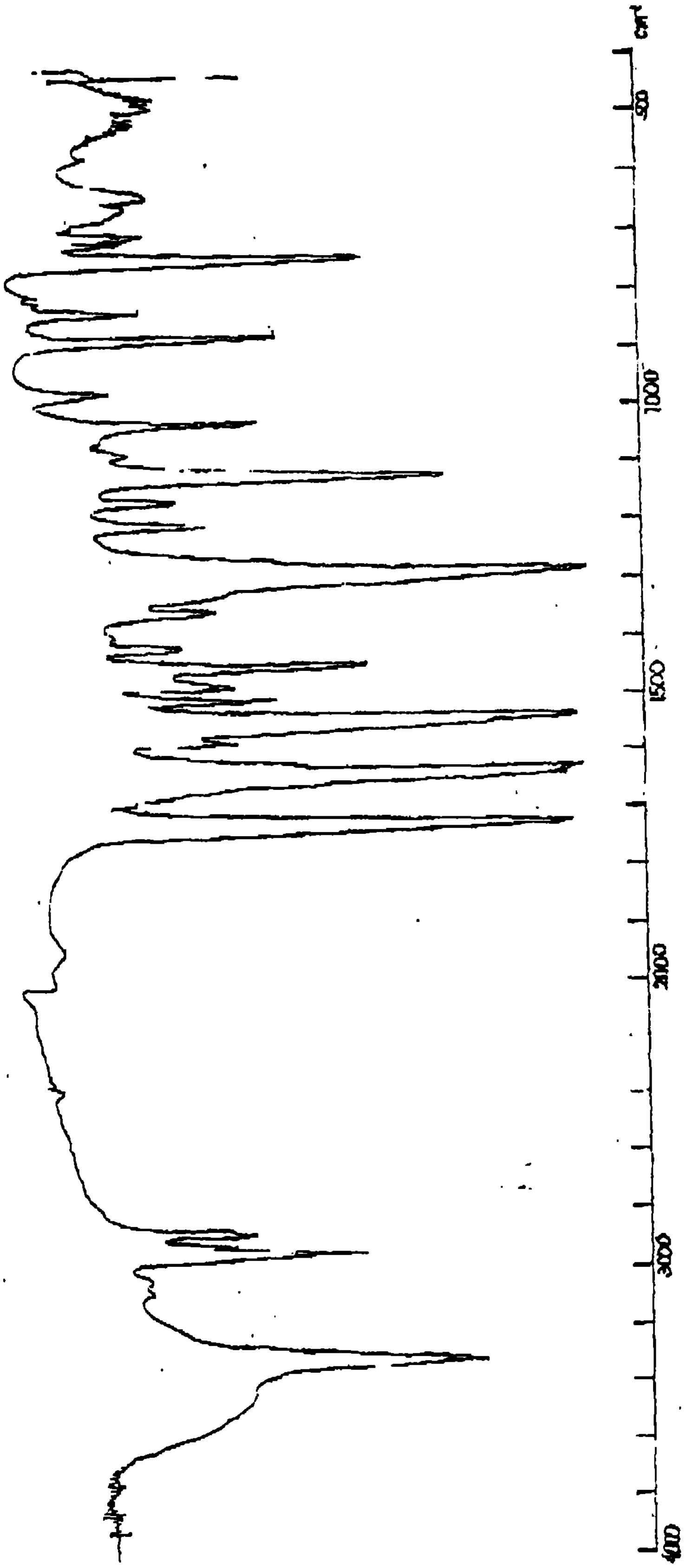

Fig. 4

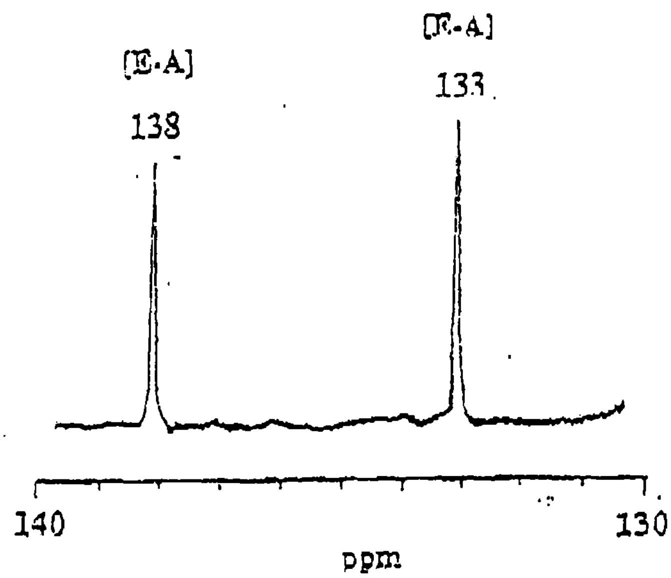


Fig. 5

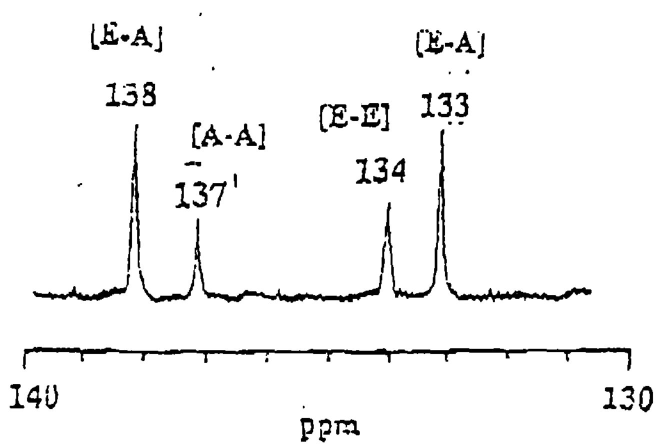

Fig. 6

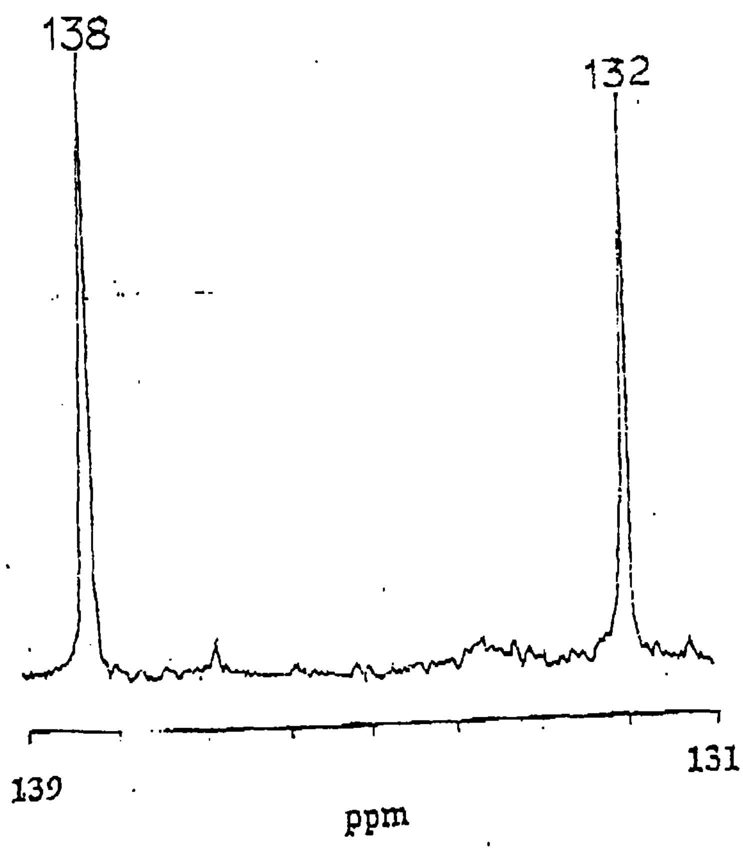

Fig. 7

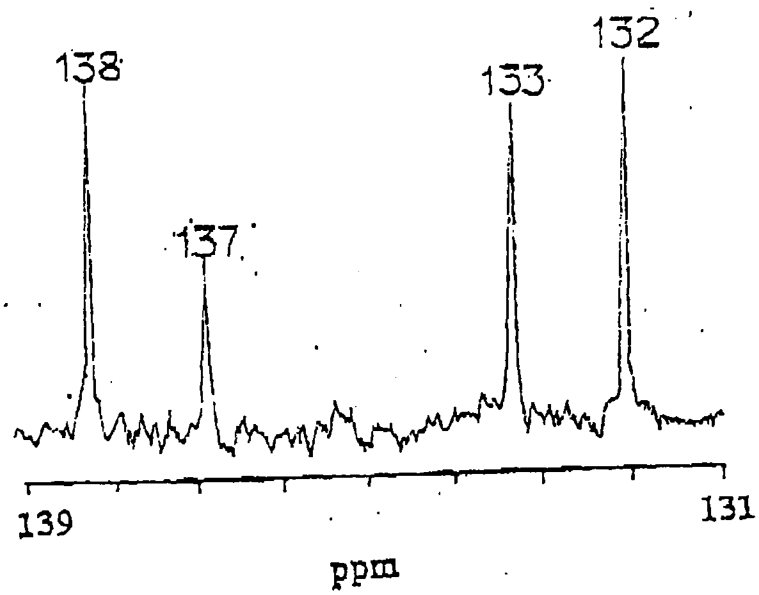

## INTERNATIONAL SEARCH REPORT

International application No. PCT/JP97/01411

**A. CLASSIFICATION OF SUBJECT MATTER**

Int. Cl[6] C08G69/44, C08L77/12, C07C233/65, C07C235/46, D01F6/82, C08J5/00, C07C233/04

According to International Patent Classification (IPC) or to both national classification and IPC

**B. FIELDS SEARCHED**

Minimum documentation searched (classification system followed by classification symbols)

Int. Cl[6] C08G69/44, C08L77/12, C07C233/65, C07C235/46, D01F6/82, C08J5/00, C07C233/04

Documentation searched other than minimum documentation to the extent that such documents are included in the fields searched

Electronic data base consulted during the international search (name of data base and, where practicable, search terms used)

CAS ONLINE
WPI/L

**C. DOCUMENTS CONSIDERED TO BE RELEVANT**

| Category* | Citation of document, with indication, where appropriate, of the relevant passages | Relevant to claim No. |
|---|---|---|
| X | JP, 56-103221, A (Toray Industries, Inc.), August 18, 1981 (18. 08. 81), Claim (Family: none) | 1-33, 51-67 |
| X | JP, 57-139122, A (Kaneka Corp.), August 27, 1982 (27. 08. 82), Claim & EP, 58938, A1 | 1-33, 51-67 |
| X | JP, 57-207621, A (Toray Industries, Inc.), December 20, 1982 (20. 12. 82), Claim & EP, 69475, A1 | 1-33, 51-67 |
| X | JP, 63-120729, A (Toray Industries, Inc.), May 25, 1988 (25. 05. 88), Claim (Family: none) | 1-33, 51-67 |
| X | JP, 4-126720, A (Kawasaki Steel Corp.), April 27, 1992 (27. 04. 92), Claim (Family: none) | 1-33, 51-67 |
| X | JP, 54-95700, A (Hitachi Chemical Co., Ltd.), | 1 - 67 |

[X] Further documents are listed in the continuation of Box C. [ ] See patent family annex.

* Special categories of cited documents:

- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| July 23, 1997 (23. 07. 97) | August 5, 1997 (05. 08. 97) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP97/01411

C (Continuation). DOCUMENTS CONSIDERED TO BE RELEVANT

| Category* | Citation of document, with indication, where appropriate, of the relevant passages | Relevant to claim No. |
|---|---|---|
| | July 28, 1979 (28. 07. 79), Claim & DE, 2900932, A1 & US, 4237251, A & FR, 2414518, A1 | |
| X | JP, 56-40649, A (ANIC S.p.A.), April 16, 1981 (16. 04. 81), Claim; example & DE, 3029970, A1 & US, 4614815, A & FR, 2468583, A1 | 1 - 67 |
| X | JP, 61-106625, A (Dainippon Ink and Chemicals, Inc.), May 24, 1986 (24. 05. 86), Claim; example (Family: none) | 1 - 67 |
| X | JP, 61-236827, A (Kuraray Co., Ltd.), October 22, 1986 (22. 10. 86), Claim (Family: none) | 1 - 67 |

Form PCT/ISA/210 (continuation of second sheet) (July 1992)